# EUROPEAN PATENT APPLICATION

(11) **EP 3 327 691 A1**
(43) Date of publication of application: **30.05.2018**
(21) Application number: 16834979.3
(22) Date of filing: 27.07.2016
(51) Int. Cl.: G08B 23/00, A61G 7/043, A61G 12/00, G08B 25/04, H04N 7/18

(54) **SYSTEM FOR MONITORING PERSON TO BE MONITORED, MONITORING INFORMATION SCREEN DISPLAY DEVICE, AND MONITORING INFORMATION SCREEN DISPLAY METHOD**

(30) Priority: 10.08.2015 JP 2015158175
(71) Applicant: Konica Minolta, Inc., Tokyo 100-7015 (JP)
(72) Inventor: NISHIKADO, Masashi, Tokyo 100-7015 (JP); NODA, Atsuhiro, Tokyo 100-7015 (JP); TSUJI, Aki, Tokyo 100-7015 (JP); YAMASHITA, Masanori, Tokyo 100-7015 (JP)
(74) Representative: Hoffmann Eitle
(86) International application number: PCT/JP2016/071999
(87) International publication number: WO 2017/026282

(57) **Abstract**

Disclosed is a monitoring information screen display device for use in a to-be-observed person monitoring system equipped with a sensor device configured to, when it judges that a to-be-observed person has performed a given action, transmit monitoring information including a result of the judgment. The monitoring information screen display device comprises a communication section, a display section, and a display processing section. The display processing section is configured to generate a monitoring information screen including the judgment result, based on the monitoring information received by the communication section, and cause the display section to display the monitoring information screen thereon. The display processing section is operable, when the communication section receives new monitoring information in a state in which the display processing section causes the display section to display the monitoring information screen thereon, to cause the display section to display thereon another monitoring information screen generated based on the new monitoring information received by the communication section, in place of the monitoring information screen currently displayed on the display section.

## Description

### TECHNICAL FIELD

The present invention relates to a to-be-observed person monitoring system for monitoring, as a person to be monitored (to-be-monitored person), a person who is subject to nursing care, e.g., and a display device and a display method applicable to this system.

### BACKGROUND ART

As a result of a rise in living standards, an improvement in hygienic environment, a rise in level of medical treatment along with rapid economic growth after World War II, and the like, Japan is entering into an aged society, more specifically, a super-aged society in which a population aging rate, i.e., a ratio of a population of persons aged 65 or older to a total population, is greater than 21%. Further, there are forecasts that, in 2020, the population of elderly persons, i.e., persons aged 65 or older, will reach about 34.56 million in a total population of about 124.11 million, while, in 2005, the population of elderly persons was about 25.56 million in a total population of about 127.65 million. In such an aged society, it is forecast that the number of persons requiring nursing care due to disease, injury, advanced age and the like becomes greater than that in a non-aged or normal society.

In addition to being a super-aged society, Japan is a society with fewer children, in which a total fertility rate, e.g., in 2013, is 1.43%. This causes problems, such as "elderly care by the elderly" which is a situation where an elderly person requiring nursing care is taken into care by his/her aged family member (spouse, child or sibling).

Such a person requiring nursing care enters or gets a place on a facility such as a hospital or a welfare facility for the aged (e.g., in Japan, a respite care facility for the aged, a nursing home for the aged or a special nursing home for the aged), and lives while receiving nursing care. However, this situation involves problems such as fall-off from a bed, fall-over during walking, and wandering after getting out of a bed. If the person is kept in any of the above conditions for a long period of time without any help, it can cause a more serious problem. Thus, in such a facility, a nurse, caregiver or the like regularly goes the round to confirm the safety or condition of the person. However, the number of persons who are willing to become caregivers cannot catch up with an increasing number of persons requiring nursing care, and therefore the nursing-care facilities industry has a problem of chronic labor shortage. Moreover, in a time zone of quasi night shift or night shift, the number of nurses, caregivers or the like is reduced, as compared to a time zone of day shift, and therefore a workload per person is increased. Thus, there is a demand for easing of the workload. Further, as regards the problem of the "elderly care by the elderly", nursing-care facilities are no exception. It is often the case that an aged caregiver takes care of an aged person requiring nursing care, and there is a nursing-care facility in which most of caregivers working for the facility are elderly. As a person ages, his/her physical strength declines, so that, as compared to young caregivers, nursing care puts a heavier burden on aged caregivers and their action and judgement inevitably become slower, even if they are healthy. In a nursing care service, quick judgement is critical for preventing accidents, such as fall-over and fall-off, and a delay in judgement can lead to a situation where it is too late to take measures. In order to ease the labor shortage and the burden on caregivers, there is a need for a technique of complementing a nursing care service. Therefore, in recent years, there has been researched and developed a to-be-observed person monitoring apparatus for monitoring a to-be-observed person, i.e., a monitoring target to be monitored, such as a person requiring nursing care.

As one example of such a technique, there is a nurse call system disclosed in the following Patent Literature 1. The nurse call system disclosed in the following Patent Literature 1 comprises a nurse-call child unit installed in a bed and configured to allow a patient to call a nurse, and a nurse-call parent unit installed in a nurse station and configured to respond to the calling from the nurse-call child unit, wherein the nurse call system further comprises: a camera configured to take an image of the patient on the bed from above the bed; and state judgment means operable to judge occurrence of at least one of a state in which the patient sits up on the bed and a state in which the patient gets out of the bed and then output an attentional-state occurrence signal, and wherein the nurse-call parent unit comprises alarming means operable to provide an alarm in response to receiving the attentional-state occurrence signal.

On the other hand, in terms of confirmation of the safety, an person living alone is a person to be monitored, as well as the person requiring nursing care or the like.

As a to-be-observed person monitoring system, assume a configuration in which, when a sensor device judges that a to-be-observed person has performed a given action, it transmits monitoring information including a result of the judgment, and, based on the monitoring information, a display device generates and displays a monitoring information screen. There is a possibility that, in a situation where a monitorer is considering handling while looking at the monitoring information screen displayed on the display device, the display device receives new monitoring information (i.e., latest monitoring information). In this case, it is necessary to prevent the monitorer from overlooking a judgment result included in the new monitoring information. In particular, when the new monitoring information includes a critical judgment result, overlooking of the judgment result can cause a worse situation, as compared to the case without the overlooking.

### CITATION LIST

### [Parent Document]

Patent Literature 1: JP 2014-90913 A

### SUMMARY OF INVENTION

It is an object of the present invention to provide a to-be-observed person monitoring system, a monitoring information screen display device and a monitoring information screen display method capable of, when new monitoring information arrives in a state in which a monitoring information screen is displayed, preventing a monitorer from overlooking a judgment result included in the new monitoring information.

According to a first aspect of the present invention, there is provided a monitoring information screen display device for use in a to-be-observed person monitoring system equipped with a sensor device which transmits monitoring information including a result of judgment when it judges that a to-be-observed person has performed a given action. The monitoring information screen display device comprises: a communication section which receives the monitoring information via a network; a display section; and a display processing section which generates a monitoring information screen including the judgment result, based on the monitoring information received by the communication section, and causes the display section to display the monitoring information screen thereon, wherein the display processing section is operable, when the communication section receives new monitoring information in a state in which the display processing section causes the display section to display the monitoring information screen thereon, to cause the display section to display thereon another monitoring information screen generated based on the new monitoring information received by the communication section, in place of the monitoring information screen currently displayed on the display section.

These and other objects, features, and advantages of the present invention will become apparent upon reading of the following detailed description along with the accompanying drawings.

### BRIEF DESCRIPTION OF DRAWINGS

FIG. 1 is a diagram depicting a configuration of a to-be-observed person monitoring system according to one embodiment of the present invention.
FIG. 2 is a diagram depicting a configuration of a mobile terminal unit in the to-be-observed person monitoring system according to this embodiment.
FIG. 3A is a diagram presenting items of a monitoring information table to be stored in the mobile terminal unit.
FIG. 3B is a diagram presenting items of a detection unit information table to be stored in the mobile terminal unit.
FIG. 4 is a diagram depicting some examples of icons to be used in the to-be-observed person monitoring system according to this embodiment.
FIG. 5 is a flowchart presenting operation regarding display of monitoring information in the mobile terminal unit.
FIG. 6 is a diagram depicting one example of a standby screen displayed on the mobile terminal unit.
FIG. 7 is a diagram depicting one example of a monitoring information screen displayed on the mobile terminal unit when it receives a notification about sitting-up of a to-be-observed person.
FIG. 8 is a diagram depicting one example of a monitoring information screen displayed on the mobile terminal unit when it receives a notification about out-of-bed of the to-be-observed person after receiving the notification about the sitting-up.
FIG. 9 is a diagram depicting one example of a monitoring information screen displayed on the mobile terminal unit in a full screen mode.
FIG. 10 is a diagram depicting one example of a monitoring information screen currently displayed on a TA display section.
FIG. 11 is a diagram depicting one example of a monitoring information screen displayed on the TA display section, in place of the monitoring information screen depicted in FIG. 10.
FIG. 12 is a diagram depicting the monitoring information table stored in a monitoring information storage section of each of a plurality of the mobile terminal units when the monitoring information screen depicted in FIG. 10 is displayed.
FIG. 13 is a diagram depicting the monitoring information table stored in the monitoring information storage section of each of the mobile terminal units when the monitoring information screen depicted in FIG. 11 is displayed.
FIG. 14 is a diagram depicting another example of the monitoring information screen displayed on the TA display section, in place of the monitoring information screen depicted in FIG. 10.
FIG. 15 is a diagram depicting one example of a monitoring information screen switched by flicking the monitoring information screen depicted in FIG. 11 upwardly.
FIG. 16 is a diagram depicting one example of a plane generated in a first modification.
FIG. 17 is a diagram depicting one example of planes generated in a second modification.
FIG. 18 is a diagram depicting one example of a plane generated in a third modification.

### DESCRIPTION OF EMBODIMENTS

Based on the drawings, one embodiment of the present invention will now be described. It should be noted that elements or components assigned with the same reference sign in the figures mean that they are identical, and therefore an already-described content of the component will be omitted.

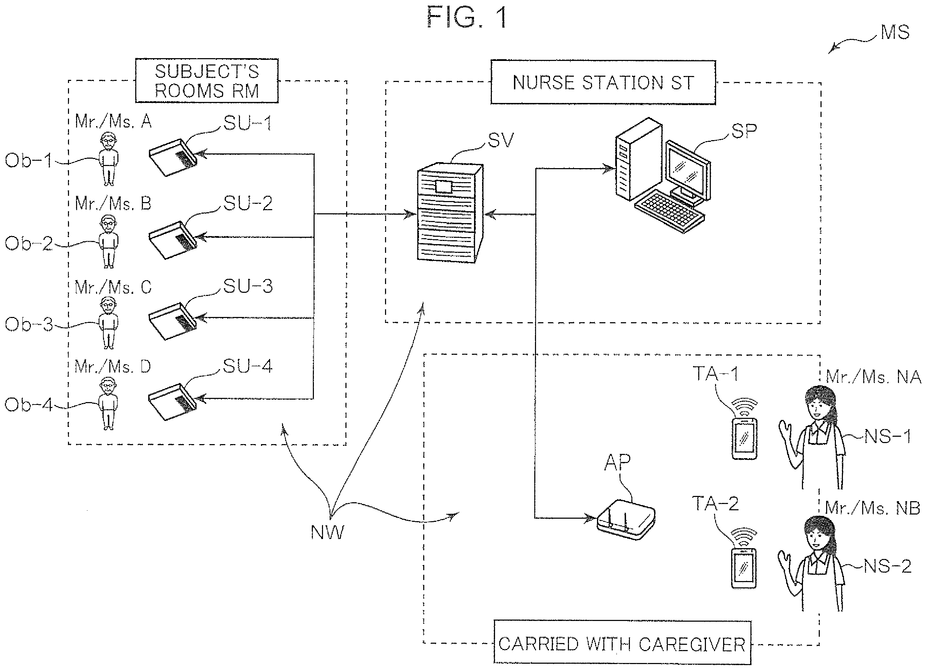

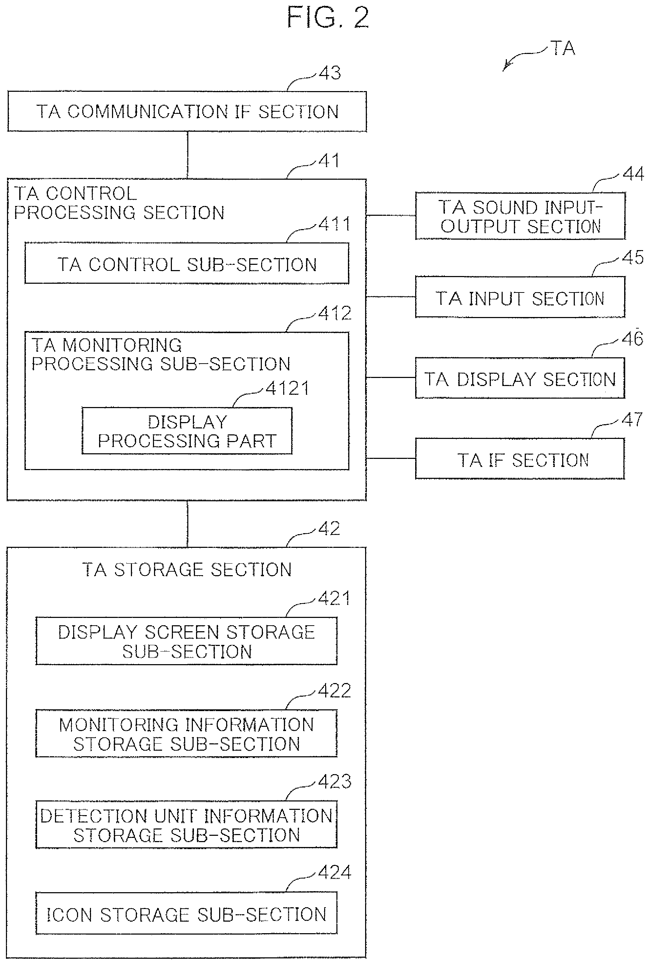

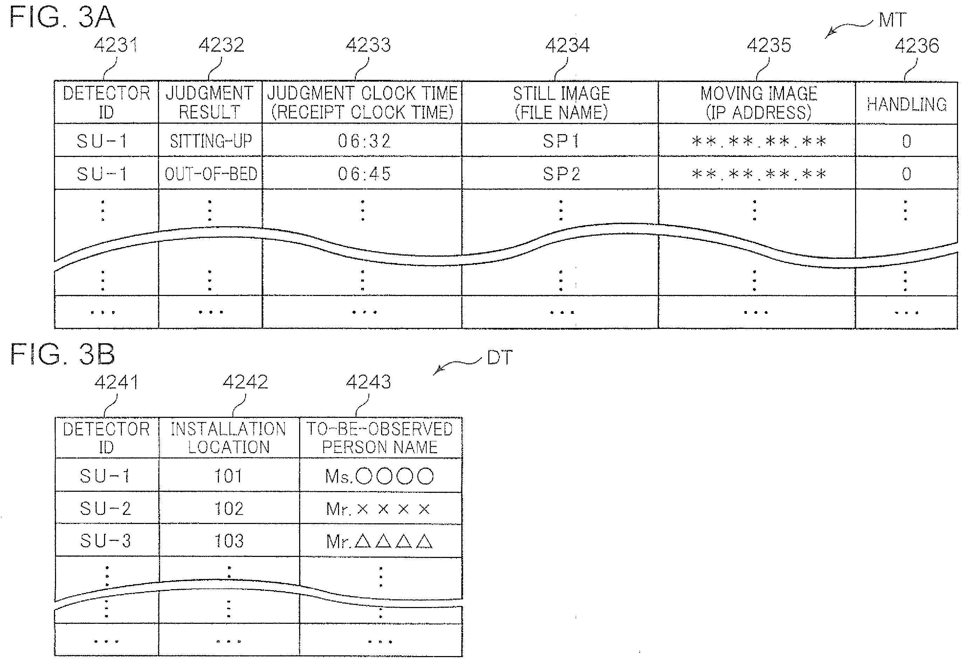

FIG. 1 is a diagram depicting a configuration of a to-be-observed person monitoring system according to one embodiment of the present invention. FIG. 2 is a diagram depicting a configuration of a mobile terminal unit in the to-be-observed person monitoring system according to this embodiment. FIG. 3A is a diagram presenting items of a monitoring information table to be stored in the mobile terminal unit. FIG. 3B is a diagram presenting items of a detection unit information table to be stored in the mobile terminal unit.

For example, as depicted in FIG. 1, the to-be-observed person monitoring system MS comprises a plurality of detection units (sensor units) SU (SU-1 to SU-4), a management server unit SV, a stationary terminal unit SP, and one or more mobile terminal units TA (TA-1, TA-2), which are communicably connected to each other in a wired or wireless manner via a network (or communication line) NW, such as a LAN (Local Area Network), a telephone network or a data communication network. The network NW may be equipped with an intermediary device for relaying a communication signal, such as a repeater, a bridge, a router or a cross-connect. In the example depicted in FIG. 1, the plurality of detection units SU-1 to SU-4, the management server unit SV, the stationary terminal unit SP and a plurality of the mobile terminal units TA-1, TA-2 are communicably connected to each other via a network NW composed of a wireless LAN (e.g., LAN according to the IEEE 802.11 standard) including an access point AP.

The to-be-observed person monitoring system MS is provided in an appropriate location depending on a to-be-observed person Ob. Examples of the to-be-observed person (watching target person) Ob include a person requiring nursing care due to disease, injury and the like, a person requiring nursing care due to a decline in physical performance or the like, and a single-living person. Particularly, from a viewpoint of enabling early detection and rapid coping, the to-be-observed person Ob is preferably a person who has to ask someone to find the occurrence of given undesirable states such as an abnormal state. Therefore, the to-be-observed person monitoring system MS is suitably provided in a building such as a hospital, a welfare facility for the aged, or a dwelling unit, depending on a type of to-be-observed person Ob. In the example depicted in FIG. 1, the to-be-observed person monitoring system MS is provided in a building of a nursing-care facility having a plurality of rooms such as two or more to-be-observed person's rooms RM each occupied by a respective one of two or more to-be-observed persons Ob, and a nurse station ST.

Each of the detection units SU is a device which has a communication function for communicating with each of the other units SV, SP, TA via the network NW, and a function of imaging a to-be-observed person Ob to generate an image thereof, and detecting the to-be-observed person Ob based on the generated image. More specifically, as one example, the detection unit SU is constructed such that it comprises: communication interface circuit (e.g., LAN card) for communicating with each of the other units SV, SP, TA via the network; a Doppler shift-type body motion sensor configured to transmit and receive a microwave and detect a Doppler shift caused in the microwave by a body motion (e.g., respiratory motion) of a to-be-observed person Ob to thereby detect detect the to-be-observed person Ob; an image sensor configured to image the to-be-observed person Ob to generate an image thereof; a data processing circuit configured to judge a state (condition) of the to-be-observed person Ob as a detection result of the to-be-observed person Ob, based on an output of the body motion sensor (body motion sensor's output) and an output (image) of the image sensor,; a control circuit for controlling the above components; and peripheral circuits of the control circuit, wherein it is operable to transmit the detection result to the management server unit SV. The detection unit SU is also operable to transmit the generated image (including a still image and a moving image) to given ones of the other units SV, SP, TA. In this embodiment, the detection unit SU comprises a nurse call circuit for notifying a nurse call to the stationary terminal unit SP, the mobile terminal units TA or the like, and a talking circuit for performing voice talk with the stationary terminal unit SP, the mobile terminal units TA or the like, whereby it is capable of performing nurse call and voice talk. As one example, FIG. 1 depicts four, first to fourth, detection units SU-1 to SU-4, wherein: the first detection unit SU-1 is provided in a to-be-observed person's room RM-1 (not depicted) of Mr./Ms. A who is one Ob-1 of the four to-be-observed persons Ob; the second detection unit SU-2 is provided in a to-be-observed person's room RM-2 (not depicted) of Mr./Ms. B who is one OB-2 of the remaining three to-be-observed persons Ob; the third detection unit SU-3 is provided in a to-be-observed person's room RM-3 (not depicted) of Mr./Ms. C who is one Ob-3 of the remaining two to-be-observed persons Ob; the fourth detection unit SU-4 is provided in a to-be-observed person's room RM-4 (not depicted) of Mr./Ms. D who is the remaining to-be-observed person Ob-4.

The management server unit SV is a device which has a communication function of communicating with each of the other units SU, TA, SP via the network NW, and a function of receiving, from each of the detection units SU, the detection result of a corresponding one of the to-be-observed persons Ob and the image of the corresponding to-be-observed person Ob, and managing information regarding monitoring (monitoring information) of the corresponding to-be-observed person Ob. The management server unit SV is operable, in response to receiving, from each of the detection units SU, the detection result of a corresponding one of the to-be-observed persons Ob and the image of the corresponding to-be-observed person Ob, to transmit a communication signal (monitoring information communication signal) containing the monitoring information of the corresponding to-be-observed person Ob, to the stationary terminal unit SP and the mobile terminal units TA. Further, the management server unit SV is operable to provide data responding to a request from a client (in this embodiment, the stationary terminal unit SP, each of the mobile terminal units TA or the like) to the client. This management server unit SV can be composed, for example, of a computer with a communication function.

The stationary terminal unit SP is a device which has a communication function of communicating with each of the other units SU, SV, TA via the network NW, and other functions such as a display function of displaying given information thereon, and an input function of accepting an input of a given instruction or data, i.e., a function of performing various operations, such as operation of accepting an input of a given instruction or data to be provided to the management server unit SV or the mobile terminal units TA, and operation of displaying thereon the detection result and the image obtained by each of the detection units SU, whereby it functions as a user interface (UI) of the to-be-observed person monitoring system MS. This stationary terminal unit SP can be composed, for example, of a computer with a communication function.

Each of the mobile terminal units TA is a device which has a communication function of communicating with each of the other units SV, SP, SU via the network NW, and other functions such as a display function of displaying given information thereon, an input function of accepting an input of a given instruction or data therethrough, and a talking function of performing voice talk, i.e., a function of performing various operations such as operation of accepting an input of a given instruction or data to be provided to the management server unit SV or the detection units SU, and operation of displaying thereon the detection result and the image obtained by each of the detection units SU, based on a notification from the management server unit SV, whereby it is capable of accepting and displaying the monitoring information of each of the to-be-observed persons Ob.

The management server unit SV is an example of the disply device and may function as the display device, or the stationary terminal unit SP is an example of the disply device and may function as the display device. However, this embodiment will be described on an assumption that each of the mobile terminal units TA functions as the display device as one example thereof. In the case where the management server unit SV functions as the display device as one example thereof, the management server unit SV can be configured in the same manner as that described above. Further, in the case where the stationary terminal unit SP functions as the display device as one example thereof, the stationary terminal unit SP can be configured in the same manner as that described above.

In this embodiment, for example, as depicted in FIG. 2, each of the mobile terminal units TA comprises a terminal control processing section (TA control processing section) 41, a terminal storage section (TA storage section) 42, a terminal communication interface section (TA communication IF section) 43, a terminal sound input-output section (TA sound input-output section) 44, a terminal input section (TA input section) 45, a terminal display section (TA display section) 46, and a terminal interface section (TA IF section) 47.

The TA sound input-output section 44 is a device connected to the TA control processing section 41, and designed to acquire external sound and input into the mobile terminal unit TA and to generate and output sound according to an electric signal indicative of the sound, under control of the TA control processing section 41. For example, the TA sound input-output section 44 is constructed such that it comprises: means to convert acoustic vibration of sound to an electric signal, such as a microphone; and means to convert an electric signal indicative of sound to acoustic vibration of the sound, such as a speaker. The TA sound input-output section 44 is operable to output an electric signal indicative of external sound to the TA control processing section 41, and to convert an electric signal input from the TA control processing section 41 to acoustic vibration of sound, and output the sound.

The TA input section 45 is a device connected to the TA control processing section 41, and designed to accept a given manipulation to accept input to the mobile terminal unit TA. It may be composed, for example, of a plurality of input switches each assigned with a given function. Examples of the given manipulation includes various manipulations necessary for monitoring, such as: a manipulation for inputting a log-in ID; a manipulation for inputting information about whether or not handling, such as nursing care, should be performed on the to-be-observed person Ob whose detection result and image has been notified; and a manipulation for inputting, into this mobile terminal unit TA, information that it is being judged (it is under a judgment as to) whether or not the handling should be performed. The TA display section 46 is a device connected to the TA control processing section 41, and designed to display, under control of the TA control processing section 41, information such as the content of the given manipulation input from the TA input section 45, and the monitoring information of each of the to-be-observed persons Ob monitored by the to-be-observed person monitoring system MS (e.g., the judged state and the image of the to-be-observed person Ob). For example, it may be a display such as an LCD or an organic EL display. In this embodiment, the TA input section 45 and the TA display section 46 are constructed as a touch panel. In this case, for example, the TA input section 45 is a certain type (e.g., resistive or capacitive type) of position input device configured to detect and input a manipulated position. This touch panel is constructed such that the position input device is provided on a display surface of the TA display section 46, wherein, when one or more inputtable candidate items are displayed on the TA display section 46, and a user (monitorer) such as a nurse or a caregiver touches a display position where a desired one of the items is displayed, the touched position is detected by the position input device, and the item displayed at the detected position is input into the mobile terminal unit TA as the content of a manual input of the user.

The TA IF section 47 is a device connected to the TA control processing section 41 and designed to perform, under control of the TA control processing section 41, data input and output with respect to an external device. Examples thereof include an interface circuit employing the Bluetooth (registered trademark) standard, an interface circuit for performing infrared communication, compliant with the IrDA (Infrared Data Association) standard or the like, and an interface circuit employing the USB (Universal Serial Bus) standard.

The TA communication IF section 43 is a communication device connected to the TA control processing section 41 and designed to perform communications under control of the TA control processing section 41. The TA communication IF section 43 is operable to generate a communication signal containing data input from the TA control processing section 41 and deemed to be transferred, according to a communication protocol used in the network NW of the to-be-observed person monitoring system MS, and transmit the generated communication signal to each of the other units SU, SV, SP via the network NW. The TA communication IF section 43 is operable to receive a communication signal from each of the other units SU, SV, SP via the network NW, and after extracting data from the received communication signal, and converting the extracted data to data having a format processable by the TA control processing section 41, output the converted data to the TA control processing section 41.

The TA storage section 42 is a circuit connected to the TA control processing section 41 and designed to store therein a variety of given programs and a variety of given data, under control of the TA control processing section 41. Examples of the variety of given programs include a control processing program such as a monitoring processing program for processing the monitoring information of each of the to-be-observed persons Ob. Examples of the monitoring processing program include a display processing program for processing operation regarding display of the monitoring information. The above variety of given data include the monitoring information of each of the to-be-observed persons Ob, and information about each of the detection units SU, i.e., detection unit information of each of the detection units SU. For example, the TA storage section 42 comprises a ROM (Read Only memory) which is a non-volatile storage element, an EEPROM (Electronically Erasable Programmable Read Only Memory) which is a rewritable non-volatile storage element) or the like. The TA storage section 42 also comprises a RAM (Random Access Memory) for storing therein data generated during execution of the given program, i.e., serving as a so-called working memory of the TA control processing section 41. Further, the TA storage section 42 functionally comprises a display screen storage section 421, a monitoring information storage section 422 and a detection unit information storage section 423.

The display screen storage section 421 is a means to store an image, such as a display screen, to be displayed on the TA display section 46, under control of an aftermentioned display processing part 4121 in the TA control processing section 41, and is composed, for example, of a VRAM (video memory). The display screen storage section 421 is operable, when there are a plurality of monitoring information screens (described in detail later) indicative, respectively, of a plurality of pieces of monitoring information of one or more of the to-be-observed persons Ob, to store the plurality of monitoring information screens in time-serially associated relation to each other. The plurality of monitoring information screens time-serially associated with each other may be displayed on the TA display section 46, in such a manner as to selectively switch from one of the monitoring information screens to another of the monitoring information screens, in response to an input manipulation accepted by the TA input section 45, or may be displayed on the TA display section 46, in such a manner as to shift one of the monitoring information screens to another of the monitoring information screens, while sequentially displaying the monitoring information screens, in response to an input manipulation accepted by the TA input section 45. More specifically, in this embodiment, when a plurality of monitoring information communication signals each containing the monitoring information of a respective one of the two or more to-be-observed persons Ob different from each other, a plurality of monitoring information screens each corresponding to a respective one of the plurality of monitoring information communication signals are time-serially coupled to each other and formed into a plane. More specifically, for example, in this embodiment, the plurality of monitoring information screens are coupled to each other in an upward-downward direction when displayed on the TA display section 46, and formed into a plane. It should be noted that the upward-downward direction may be changed to a rightward-leftward direction. For example, the time-series order may be an order according to a clock time at which each of the monitoring information communication signals is received, or may be an order according to a clock time at which each of the aforementioned judgments is made. A size of the plane stored in the display screen storage section 421 is normally equal to a size of a screen display region of the TA display section 46. However, when a plurality of the monitoring information communication signals are received, a monitoring information screen for displaying the monitoring information contained in one monitoring information communication signal is formed into a plane having the normal size, and a plurality of monitoring information screens corresponding to the plurality of monitoring information communication signals are time-serially coupled to each other and formed into a plane, so that the plane formed when the plurality of monitoring information communication signals are received has a size proportional to the number of monitoring information screens. Among the plurality of monitoring information screens formed into the plane, only a part conforming to the size of the screen display region of the TA display section 46 is displayed on the TA display section 46 according to an input operation accepted by the TA input section 45, under control of the aftermentioned display processing part 4121.

The monitoring information storage section 422 is a means to store the monitoring information of each of the to-be-observed persons Ob. The monitoring information includes: a plurality of detection unit identifiers (detector IDs) for specifying and identifying the detection units SU, respectively; information indicative of a result of judgment made by the detection unit SU having one of the detector IDs associated with this information, as the state of the to-be-observed person Ob monitored by this detection unit SU (judgment result information; in this embodiment, including "sitting-up", "out-of-bed", "fall-over/fall-off', and "micro body motion abnormality"); information indicative of a clock time at which the detection unit SU having one of the detector IDs associated with this information judges the state of the to-be-observed person Ob monitored by this detection unit SU (judgment clock time information); a still image of the to-be-observed person Ob monitored by the detection unit SU having one of the detector IDs associated with the still image and used when this detection unit SU judged the state of the monitored to-be-observed person Ob (in the case where the judgment is made using a plurality of images, one (e.g., a latest one) of the images is selected); a communication address (e.g., IP address) of the detection unit SU having one of the detector IDs associated with the communication address; and handling information indicative of whether an intention to perform handling, such as life-saving action, nursing care and assistance, on the to-be-observed person Ob monitored by the detection unit SU having one of the detections ID associated with the handling information, is input. It should be noted that, in place of the judgment clock time, it is possible to use a communication signal receipt clock time at which the notification of the judgment result and the image is received.

In this embodiment, the monitoring information is stored in the monitoring information storage section 422 in a table format. For example, as depicted in FIG. 3A, the monitoring information table MT has: a detector ID field 4231 for registering the detector ID thereon; a judgment result field 4232 for registering thereon the judgment result information related to the detection unit SU corresponding to the detector ID registered on the detector ID field; a judgment clock time field 4233 for registering thereon the judgment clock time information related to the detection unit SU corresponding to the detector ID registered on the detector ID field; a still image field 4234 for registering thereon the still image related to the detection unit SU corresponding to the detector ID registered on the detector ID field; a moving image field 4235 for registering thereon the communication address (e.g., IP address) of the detection unit SU corresponding to the detector ID registered on the detector ID field; and a handling field 4236 for registering thereon the handling information indicative of the presence or absence of input of the intention to perform the handling on the to-be-observed person Ob monitored by the detection unit SU corresponding to the detector ID registered on the detector ID field, wherein a record is generated with respect to each of the detector IDs. On the handling field 4236, a flag representing the handling information indicative of the presence or absence of input of the intention of the handling is registered. For example, in this embodiment, a flag "1" denoting that the intention of the handling has been input into the mobile terminal unit TA, or a flag "0" denoting that no intention of the handling has been input into the mobile terminal unit TA, is registered on the handling field 4236. On the still image field 4234, for example, image data about a still image may be registered, or a file name of image data about a still image may be registered. In the example depicted in FIG. 3A, as a record in a first row, "SU-1", "Sitting-up", "06:32", "SP1", "** ** ** **" ("**" denotes an integer), and "0" are registered, respectively, on the detector ID field 4231, the judgment result field 4232, the judgment clock time field 4233, the still image field 4234, the moving image field 4235 and the handling field 4236, and, as a record in a second row, "SU-1", "Out-of-bed", "06:45", "SP2", "** ** ** **" ("**" denotes an integer), and "0" are registered, respectively, on the detector ID field 4231, the judgment result field 4232, the judgment clock time field 4233, the still image field 4234, the moving image field 4235 and the handling field 4236.

In the example depicted in FIG. 3A, the monitoring information table MT is configured to have the moving image field 4235. Alternatively, a table presenting a correspondence relationship between respective ones of the detections ID and respective ones of the communication addresses of the detection units SU each serving as a source for acquisition of a live moving image may be prepared separately from the monitoring information table MT and stored in the monitoring information storage section 422, and the moving image field 4235 may be omitted from the monitoring information table MT depicted in FIG. 3A.

The detection unit information storage section 423 is a means to store the detection unit information of each of the detection units SU. The detection unit information includes an installation location of each of the detection units SU, and a name of the to-be-observed person monitored by each of the detection units SU. In this embodiment, the detection unit information is stored in the detection unit information storage section 423 in a table format. For example, as depicted in FIG. 3B, a detection unit information table DT for registering the detection unit information thereon has: a detector ID field 4241 for registering the detector ID thereon; an installation location field 4242 for registering an installation location of the detection unit SU having one of the detector IDs associated with the installation location; and a to-be-observed person name field 4243 for registering the name of the to-be-observed person Ob monitored by the detection unit SU having one of the detector IDs associated with the name of the monitored to-be-observed person Ob, wherein a record is generated with respect to each of the detector IDs. In the example depicted in FIG. 3B, as a record in a first row, "SU-1", "101" and "Ms. ○○○○" are registered, respectively, on the detector ID field 4241, the installation location field 4242 and the to-be-observed person name field 4243, and, as a record in a second row, "SU-2", "102" and "Mr. ××××" are registered, respectively, on the detector ID field 4241, the installation location field 4242 and the to-be-observed person name field 4243.

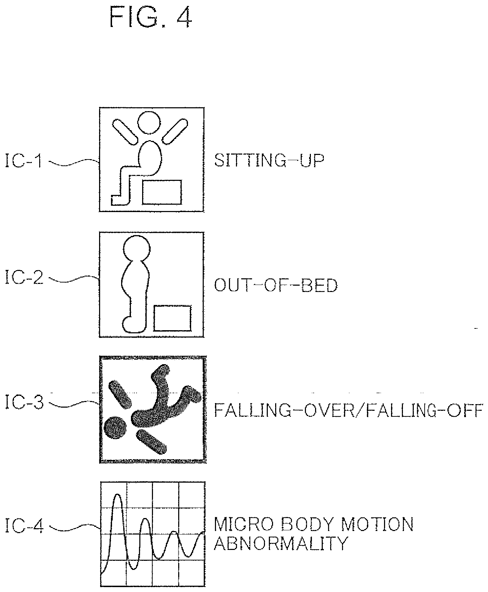

An icon storage section 424 is a means to store an icon symbolically denoting the judgment result, in associated with the judgment result. More specifically, the icon storage section 424 stores therein a plurality of icons denoting a plurality of different types of judgment results, in association with the judgment results, respectively. For example, in this embodiment, one of "sitting-up", "out-of-bed", "fall-over/fall-off' and "micro body motion abnormality" can be discriminatingly judged as the state of the to-be-observed person Ob by the detection unit SU, and (image data about) a plurality of icons each corresponding to a respective one of the judgment results ("sitting-up", "out-of-bed", "fall-over/fall-off' and "micro body motion abnormality") are preliminarily prepared. For example, as depicted in FIG. 4, the following four icons IC are preliminarily prepared: a sitting-up icon IC-1 symbolically denoting that the to-be-observed person Ob has been judged to sit up on a bed; an out-of-bed icon IC-2 symbolically denoting that the to-be-observed person Ob has been judged to move out of the bed; a fall-over/fall-off icon IC-3 symbolically denoting that the to-be-observed person Ob has been judged to fall over or fall off from the bed; and a micro body motion abnormality icon IC-4 symbolically denoting that the to-be-observed person Ob has been judged to be abnormal in micro body motion. Each of the icons is a stored in the icon storage section 424, in association with a corresponding one of the judgment results. For example, a plurality of files (electronic files) each storing a respective one of a plurality of pieces of data about the icons, and a table (icon table) presenting a correspondence relationship between respective ones of the judgment results and respective ones of file names of the icons symbolically denoting the judgment results, are preliminarily stored in the icon storage section 424.

The TA control processing section 41 is a circuit for controlling the sections of the mobile terminal unit TA depending respective functions of the sections, to process the monitoring information of each of the to-be-observed persons Ob. For example, the TA control processing section 41 is constructed such that it comprises a CPU (Central Processing Unit) and a peripheral circuit thereof. During execution of the control processing program, the TA control processing section 41 functionally comprises a terminal control section (TA control section) 411 and a terminal monitoring processing section (TA monitoring processing section) 412, wherein the TA monitoring processing section 412 functionally comprises a display processing part 4121.

The TA control section 411 is a means to control the sections of the mobile terminal unit TA depending respective functions of the sections, to govern the entire control of the mobile terminal unit TA.

The TA monitoring processing section 412 is a means to process the monitoring information of each of the to-be-observed persons Ob. More specifically, the TA monitoring processing section 412 is operable, when the TA communication IF section 43 receives the monitoring information communication signal, to register, on the monitoring information table MT, the monitoring information of the to-be-observed person Ob contained in this received monitoring information communication signal, to thereby store the monitoring information in the monitoring information storage section 422.

The display processing part 4121 is a means to process operation regarding display of the monitoring information.

The mobile terminal unit TA can be composed, for example, of a portable communication terminal device such as a so-called tablet computer, a smartphone or a mobile phone.

Next, operation of the to-be-observed person monitoring system MS according to this embodiment will be described. In the to-be-observed person monitoring system MS configured as above, upon powered on, each of the units SU, SV, SP, TA performs initialization of necessary sections, and starts to operate. In the mobile terminal unit TA, upon execution of the control processing program, the TA control section 411 and the TA monitoring processing section 412 are functionally formed in the TA control processing section 41, and further the display processing part 4121 is functionally formed in the TA monitoring processing section 412.

Generally, the to-be-observed person monitoring system MS configured as above monitors each of the to-be-observed persons Ob in the following manner. Each of the detection units SU samples an output of the body motion sensor and an output of the image sensor, in given sampling periods, and judges the state (condition) of the monitored to-be-observed person Ob, based on the sampled outputs of the body motion sensor and the image sensor, wherein, as a result of the judgment, when the detection unit SU judges that the monitored to-be-observed person Ob is in the predetermined state (e.g., in this embodiment, one of "sitting-up", "out-of-bed", "fall-over/fall-off', and "micro body motion abnormality"), the detection unit SU transmits, to the management server unit SV via the network NW, a communication signal (monitoring information communication signal) containing information, such as the detector ID of the detection unit SU, the judgment result information indicative of the judgment result judged as the state of the monitored to-be-observed person Ob, the judgment clock time information indicative of a clock time of the judgment, image data about a still image of the monitored to-be-observed person Ob used in the judgment (in the case where the judgment is made using a plurality of images, one (e.g., a latest one) of the images is selected), and a communication address (e.g., IP address) of the detection unit SU as contact information for downloading a moving image. As the communication address of of the detection unit SU, a sender's communication address contained in a header of the communication signal may be additionally used. The detection unit SU can judge the state (condition) of the monitored to-be-observed person Ob by a heretofore-known technique. For example, the detection unit SU detects a chest movement (up-and-down motion of the chest) along with a respiratory motion of the monitored to-be-observed person, by the body motion sensor, and judges that the micro body motion abnormality occurs when it detects disturbance in period of the movement of the chest, or an amplitude of the movement of the chest equal to or less than a preset threshold. Further, for example, the detection unit SU acquires an image of the monitored to-be-observed person Ob by the image sensor to detect a person region of the monitored to-be-observed person Ob from the acquired image and determines a posture (e.g., a standing posture, a sitting posture or a lying posture) based on an aspect ratio (vertical-to-horizontal ratio) of the detected person region, and then discriminatingly judges one of the "sitting-up", "out-of-bed" and "fall-over/fall-off'.

Upon receiving the monitoring information communication signal from the detection unit SU via the network NW, the management server unit SV stores (records) the information contained in the monitoring information communication signal, i.e., the detector ID, the judgment result information, the judgment clock time information, image data about a still image, and a communication address for obtaining a moving image, in associated relation to each other. Then, the management server unit SV transmits the monitoring information communication signal toward the stationary terminal unit SP and the mobile terminal units TA, e.g., in a broadcasting or multicasting manner. In this way, the state (condition) of the monitored to-be-observed person Ob is notified. In this case, when the monitoring information communication signal is transferred by the management server unit SV, the sender's communication address is changed to a communication address of the management server unit SV. Thus, in the case where the sender's communication address is additionally used as the communication address for obtaining a moving image, the management server unit SV inserts the communication address of the detection unit SU, i.e., a sender, contained in the header of the monitoring information communication signal, into a payload of the monitoring information communication signal, and then broadcasts the monitoring information communication signal. Further, the management server unit SV may transmits the monitoring information communication signal only to one or more of the mobile terminal units TA preliminarily associated with the detection unit SU in a unicasting manner.

Upon receiving the monitoring information communication signal from the management server unit SV via the network NW, the stationary terminal unit SP and each of the mobile terminal units TA displays thereon the monitoring information of the monitored to-be-observed persons Ob contained in the monitoring information communication signal. Operation of displaying this the monitoring information by the mobile terminal unit TA will be described in detail later. Based on the above operation, the to-be-observed person monitoring system MS generally detects and monitors the to-be-observed persons Ob by the detection units SU, the management server unit SV, the stationary terminal unit SP and the mobile terminal units TA.

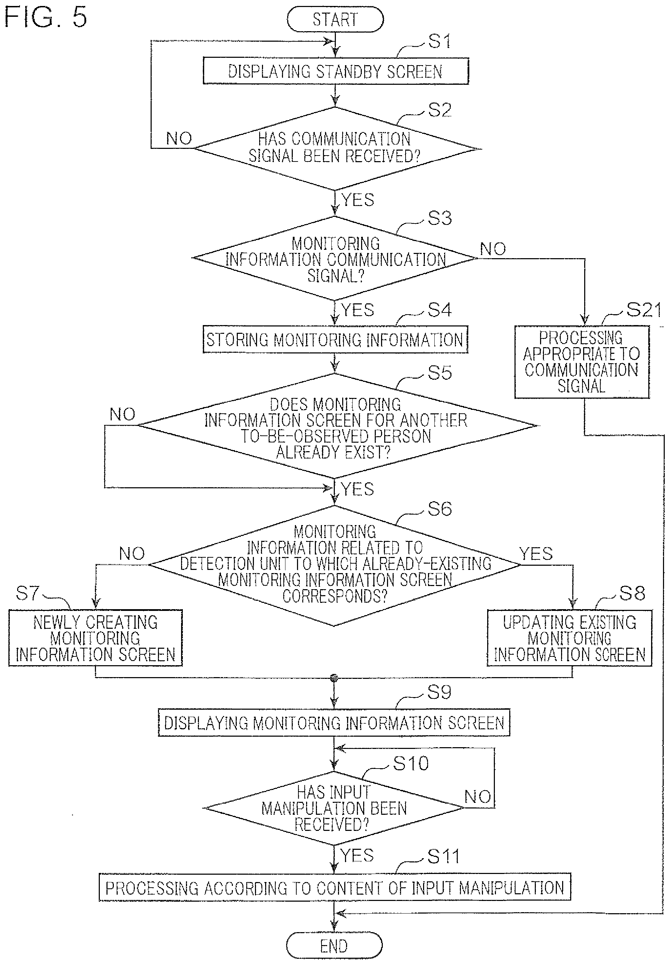

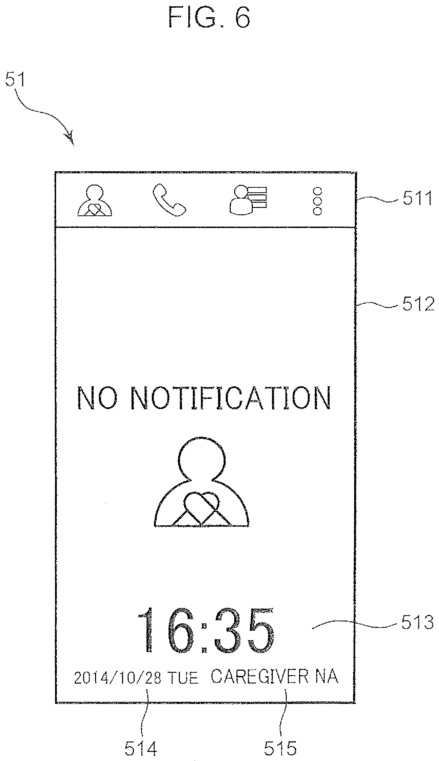

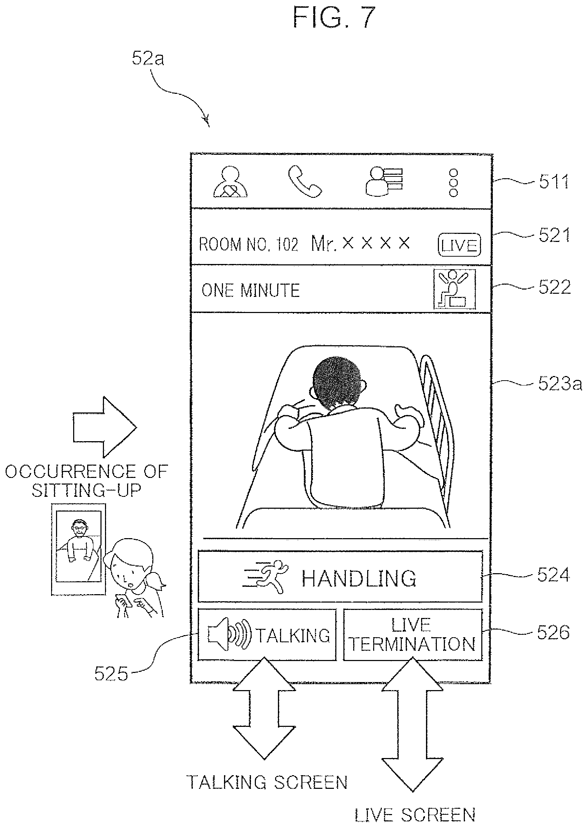

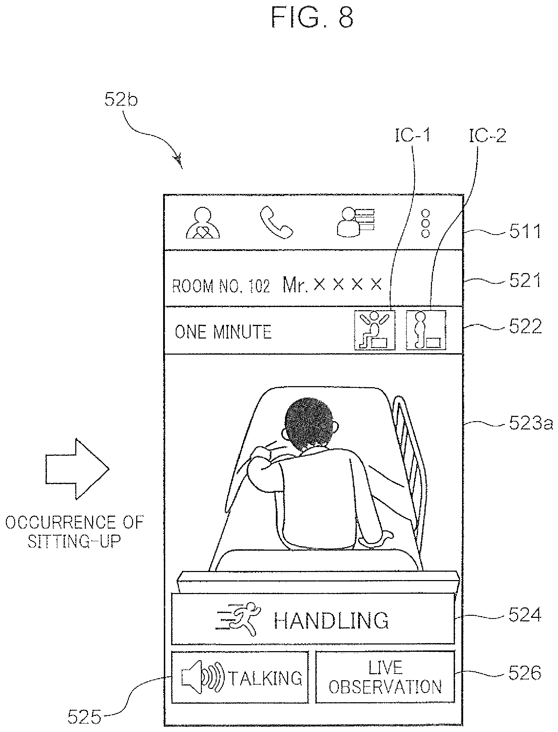

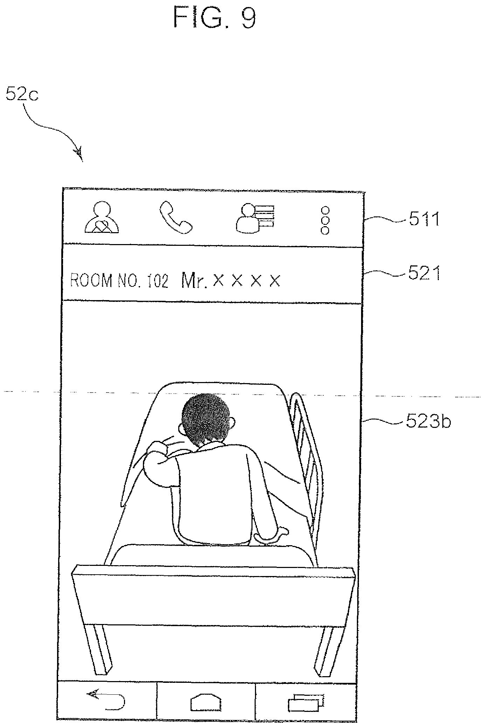

Next, the operation of displaying the monitoring information of each of the to-be-observed persons Ob in the to-be-observed person monitoring system MS will be described. FIG. 5 is a flowchart presenting operation regarding display of the monitoring information in the mobile terminal unit TA. FIG. 6 is a diagram depicting one example of a standby screen displayed on the mobile terminal unit TA. FIG. 7 is a diagram depicting one example of a monitoring information screen displayed on the mobile terminal unit TA when it receives a notification about sitting-up of the to-be-observed person Ob. FIG. 8 is a diagram depicting one example of a monitoring information screen displayed on the mobile terminal unit TA when it receives a notification about out-of-bed of the to-be-observed person Ob after receiving the notification about the sitting-up. FIG. 9 is a diagram depicting one example of a monitoring information screen displayed on the mobile terminal unit TA in a full screen mode.

In FIG. 5, when the mobile terminal unit TA after being powered-on and activated accepts a login manipulation by a monitorer (user) such as a nurse or a caregiver, the TA monitoring processing section 412 causes the TA display section 46 to display therein a standby screen for waiting for a communication signal addressed to the mobile terminal unit TA (S1). For example, as depicted in FIG. 6, this standby screen 51 has: a menu bar area 511 for displaying a menu bar thereon; a standby main area 512 for displaying thereon a message indicative of a standby state (e.g., "NO NOTIFICATION") and a related icon; a clock time area 513 for displaying a current clock time thereon; a date and day-of-week area 514 for displaying year, month, day and day-of-week of today thereon; and a user name area 515 for displaying thereon a name of a user who currently logs in to this mobile terminal unit TA.

Subsequently, the mobile terminal unit TA operates such that the TA control section 411 determines whether or not a communication signal has been received by the TA communication IF section 43 (S2). As a result of this determination, when no communication signal has been received (NO), the mobile terminal unit TA returns the processing to S1. On the other hand, as a result of this determination, when a communication signal has been received (YES), the mobile terminal unit TA executes the next processing S3.

In the processing S3, upon receiving a communication signal, the mobile terminal unit TA operates such that the TA monitoring processing section 412 determines whether or not the received communication signal is the monitoring information communication signal. As a result of this determination, when the received communication signal is not the monitoring information communication signal (NO), the mobile terminal unit TA operates such that the TA control processing section 41 executes appropriate processing according to the received communication signal (S21), and then terminates the monitoring information display operation. On the other hand, as a result of this determination, when the received communication signal is the monitoring information communication signal (YES), the mobile terminal unit TA operates such that the TA monitoring processing section 412 registers, on the monitoring information table MT, the detector ID, the judgment result information, the judgment clock time information, image data about a still image, and a communication address for obtaining a moving image, each contained in the received monitoring information communication signal, to thereby store the monitoring information in the monitoring information storage section 422 in associated relation to each other (S4), and then executes the next processing S5. When the set of information is registered on the monitoring information table MT, the TA monitoring processing section 412 registers the flag "0" on the handling field 4236 in a row of the set of information. As previously mentioned, the flag "0" means that no intention of the handling has been input into the mobile terminal unit TA. That is, the flag "0" means that a "handling" button 524 (FIG. 7) has not been touched in any of the mobile terminal units TA

In the processing S5, the mobile terminal unit TA operates such that the display processing part 4121 of the TA monitoring processing section 412 determines whether or not another monitoring information screen 52 (52-A) for displaying the monitoring information of the to-be-observed person Ob-A different from the to-be-observed person Ob-B related to the monitoring information contained in the received monitoring information communication signal already exists therein, and executes the next processing S6. More specifically, the display processing part 4121 of the TA monitoring processing section 412 determines whether or not a record having a first relationship with the detector ID contained in the received monitoring information communication signal is registered in the monitoring information table MT depicted in FIG. 3A, to thereby determine whether or not another monitoring information screen 52-A already exists. The record having the first relationship includes the detector ID different from the detector ID contained in the received monitoring information communication signal, and includes the flag "0". The term" record" has already been explained by taking the record in the first row and the record in the second row as an example.

As a result of this determination, when no record having the first relationship with the detector ID contained in the received monitoring information communication signal is registered in the monitoring information table MT, it is determined that no monitoring information screen 52-A corresponding to the different detector ID exists (NO), and the mobile terminal unit TA executes the next processing S6. On the other hand, when a record having the first relationship with the detector ID contained in the received monitoring information communication signal is registered in the monitoring information table MT, it is determined that the monitoring information screen 52-A corresponding to the different detector ID already exists (YES), and the mobile terminal unit TA executes the next processing S6. The monitoring information screen will be described in detail later.

In this processing S6, the mobile terminal unit TA operates such that the display processing part 4121 determines whether or not the monitoring information contained in the received monitoring information communication signal is information from the detection unit SU to which an already-existing monitoring information screen 52 corresponds. More specifically, the display processing part 4121 determines whether or not a monitoring information communication signal containing the detector ID identical to the detector ID contained in the received monitoring information communication signal had already (previously) been received before the received monitoring information communication signal, and a monitoring information screen 52 for the already-received monitoring information communication signal has been created for display. In other words, the display processing part 4121 determines whether or not a record having a second relationship with the detector ID contained in the received monitoring information communication signal is registered in the monitoring information table MT, to thereby determine whether or not the signal had already been received, and a monitoring information screen 52 for the already-received monitoring information communication signal has been created for display. The record having the second relationship includes the detector ID identical to the detector ID contained in the received monitoring information communication signal, and includes the flag "0".

As a result of this determination, when no record having the second relationship with the detector ID contained in the received monitoring information communication signal is not registered in the monitoring information table MT, it is determined that no monitoring information communication signal corresponding to the identical detector ID has not already been received (NO), and the mobile terminal unit TA executes processing S7. On the other hand, as a result of determination, when a record having the second relationship with the detector ID contained in the received monitoring information communication signal is registered in the monitoring information table MT, it is determined that a monitoring information communication signal corresponding to the identical detector ID has already been received (YES), and the mobile terminal unit TA executes processing S8. As will be described later in feature 1 of the to-be-observed person monitoring system MS according to this embodiment, in any of the two cases where the determination result in the processing S5 is YES and NO, the determination result in the processing S6 can be any of YES and NO.

In the processing S7, the mobile terminal unit TA operates such that the display processing part 4121 newly forms a monitoring information screen 52 (52-B) conforming to the information (data) contained in the received monitoring information communication signal, and stores it in the display screen storage section 421.

This monitoring information screen 52 is a screen for displaying the monitoring information of each of the to-be-observed persons Ob. For example, as depicted in FIG. 7, the monitoring information screen 52 (52a) has: a menu bar area 511; a to-be-observed person name area 521 for displaying thereon an installation location of the detection unit SU having a specific one of the detector IDs and a name of the to-be-observed person Ob monitored by the detection unit SU having the specific detector ID; an icon area 522 for displaying thereon an icon symbolically denoting an elapsed time from the judgment clock time (or receipt clock time) and the judgment result; an image area 523a for displaying thereon an image (in this example, a still image) taken by the detection unit SU having the specific detector ID; a "handling" button 524; a "talking" button 525; and "LIVE observation" button 526. The "handling" button 524 is a button for inputting, into the mobile terminal unit TA, information that a user of this mobile terminal unit TA has an intention to perform the handling, such as life-saving action, nursing care and assistance, on the to-be-observed person Ob monitored by the detection unit SU having the specific detector ID, and an instruction for notifying the stationary terminal unit SP and the remaining mobile terminal units TA of the fact that the intention has been input. The "talking" button 525 is a button for inputting an instruction for connecting the detection unit SU having the specific detector ID to the mobile terminal unit TA via the network NW, in a talkable manner.

In order to create the monitoring information screen 52-B conforming to the information contained in the received monitoring information communication signal, the display processing part 4121 retrieves an installation location and a to-be-observed person name corresponding to the detector ID contained in the received monitoring information communication signal, from the detection unit information storage section 423, using this detector ID as a retrieval key, and derives an elapsed time from the judgment clock time contained in the received monitoring information communication signal. Further, the display processing part 4121 retrieves an icon IC corresponding to the judgment result contained in the received monitoring information communication signal, from the icon storage section 424, using this judgment result as a retrieval key. As mentioned above, the icon storage section 424 preliminarily stores therein a plurality of icons each corresponding to a respective one of the judgment results. Then, the display processing part 4121 forms a monitoring information screen 52a in the display screen storage section 421 by: displaying a menu bar in the menu bar area 511; displaying the retrieved installation location and to-be-observed person name in the to-be-observed person name area 521; displaying the derived elapsed time and the retrieved icon IC in the icon area 522; displaying the image (still image) contained in the newly-received monitoring information communication signal, in the image area 523a; and displaying the "handling" button 524, the "talking" button 525, and the "LIVE observation" button 526, and stores the formed monitoring information screen 52a in the display screen storage section 421.

Assume that the determination in the processing S5 is made such that the monitoring information screen 52-A corresponding to the different detector ID already exists. In this case, after forming the new monitoring information screen 52-B in the above manner, the display processing part 4121 stores the newly-formed monitoring information screen 52-B and the already-existing monitoring information screen 52-A in the display screen storage section 421, in time-serially associated relation to each other. More specifically, the display processing part 4121 time-serially couples the newly-formed monitoring information screen 52-B and the already-existing monitoring information screen 52-A together in the upward-downward direction when displayed on the TA display section 46, to form a plane.

Returning to FIG. 5, in the processing S8, the mobile terminal unit TA operates such that the display processing part 4121 updates the monitoring information screen 52 (52-B) conforming to the information (data) contained in the received monitoring information communication signal, and forms and stores the updated monitoring information screen in the display screen storage section 421.

In order to update the monitoring information screen 52-B in conformity to the information contained in the received monitoring information communication signal, the display processing part 4121 derives an elapsed time from the judgment clock time contained in the received monitoring information communication signal, and retrieves an icon IC corresponding to the judgment result contained in the received monitoring information communication signal, from the icon storage section 424, using this judgment result as a retrieval key. Then, the display processing part 4121 updates the monitoring information screen 52-B by: displaying the derived elapsed time and the retrieved icon IC in the already-existing monitoring information screen 52; and displaying the image (still image) contained in the received monitoring information communication signal, in the image area 523a, and stores (forms) the updated monitoring information screen in the display screen storage section 421. In this case, the icon IC corresponding to the judgment result contained in a previously-received monitoring information communication signal is displayed in the icon area 522. Thus, the icon IC retrieved this time is displayed in a state in which it is time-serially disposed with respect to the already-displayed icon IC. For example, in the case where, after the judgment result "sitting-up" of the monitored to-be-observed person Ob is notified, the judgment result "out-of-bed" of of the monitored to-be-observed person Ob is notified, a monitoring information screen 52b depicted in FIG. 8 in which the "sitting-up" icon IC-1 denoting the judgment result "sitting-up" of the monitored to-be-observed person Ob is displayed in the icon area 522 is updated by displaying the out-of-bed icon IC-2 denoting the judgment result "out-of-bed" of of the monitored to-be-observed person Ob in the icon area 522 in a state in which it is disposed next to the sitting-up icon IC-1 denoting the judgment result "sitting-up" of the monitored to-be-observed person Ob, on the left.

Assume that the determination in the processing S5 is made such that the monitoring information screen 52-A corresponding to the different detector ID already exists. In this case, after updating and forming the monitoring information screen 52-B in the above manner, the display processing part 4121 stores the updatingly-formed monitoring information screen 52-B and the already-existing monitoring information screen 52-A in the display screen storage section 421, in time-serially associated relation to each other, in the same manner as that in the processing S7. More specifically, the display processing part 4121 time-serially couples the updatingly-formed monitoring information screen 52-B and the already-existing monitoring information screen 52-A together in the upward-downward direction when displayed on the TA display section 46, to form a plane. The display processing part 4121 may be configured to time-serially associate these screens with each other while maintaining an existing time-series association (time-series coupling), or may be configured to reorganize these screens by associating them with each other in a new time-series relationship based on the received monitoring information communication signal.

After completion of the processing S7, the display processing part 4121 causes the TA display section 46 to display the resulting monitoring information screen, e.g., the monitoring information screen 52a depicted in FIG. 7. On the other hand, after completion of the processing S8, the display processing part 4121 causes the TA display section 46 to display the resulting monitoring information screen, e.g., the monitoring information screen 52b depicted in FIG. 8 (S9).

Subsequently, the mobile terminal unit TA operates such that the TA control processing section 41 determines whether or not an input manipulation has been accepted by the touch panel comprised of the TA input section 45 and the TA display section 46 (S10). As a result of this determination, when no input manipulation has been accepted (NO), the mobile terminal unit TA returns the processing to the processing S10. On the other hand, as a result of this determination, when an input manipulation has been accepted, the mobile terminal unit TA executes the next processing S11.

In the processing S11, the mobile terminal unit TA performs appropriate processing according to the content of the input operation, and then terminates the monitoring information display operation.

For example, the mobile terminal unit TA operate such that, when "tap" is accepted as the input operation by the touch panel comprised of the TA input section 45 and the TA display section 46, the TA control processing section 41 displays a monitoring information screen 52c in which the image area 523a is changed to a full-screen image area 523b, on the TA display section 46. For example, as depicted in FIG. 9, the monitoring information screen 52c has: a menu bar area 511; a to-be-observed person name area 521; and a full-screen image area 523b for displaying an image (in this example, a still image) taken by the detection unit SU having the specific detector ID, in the entire remaining area other than the menu bar area 511 and the to-be-observed person name area 521. Based on this full-screen display, the monitorer (user) can recognize the state of the monitored to-be-observed person Ob in more detail.

For example, the mobile terminal unit TA operates such that, when an input operation of the "handling" button 524 is accepted, the TA control processing section 41 registers a flag" 1" on the handling field 4236 in a row of a record in which the detector ID corresponding to the monitoring information of the to-be-observed person Ob currently displayed on the TA display section 46 is registered on the detector ID field 4231, while notifying the stationary terminal unit SP and the remaining mobile terminal units TA of the fact that the monitorer (user) logins to this mobile terminal unit TA has an intention to perform the handling, such as nursing, on the to-be-observed person Ob currently displayed on the TA display section 46, to delete the monitoring information screen 52 corresponding to the to-be-observed person Ob displayed on the TA display section 46.

Further, for example, the mobile terminal unit TA operates such that, when an input operation of the "talking" button 525 is accepted, the TA control processing section 41 establishes a connection with the detection unit SU for detecting the to-be-observed person Ob currently displayed on the TV display section 46, via the network NW in a talkable manner.

Further, for example, the mobile terminal unit TA operates such that, when an input operation of the "LIVE observation" button 526 is accepted, the TA control processing section 41 establishes a connection with the detection unit SU for monitoring the to-be-observed person Ob currently displayed on the TV display section 46, via the network NW in a moving image-downloadable manner. In this case, a moving image of the monitored to-be-observed person Ob is displayed in the image area 523a or the image area 523b switched in response to the "tap".

Features 1 to 6 of the to-be-observed person monitoring system MS according to this embodiment will be described. Referring to FIG. 1, the to-be-observed person monitoring system MS comprises the plurality of detection units SU assigned, respectively, to the plurality of to-be-observed persons Ob. Each of the detection units SU is a specific example of a sensor device, and is operable, when it judges that a respective one of the to-be-observed persons Ob has performed a given action, to transmit the monitoring information including a result of the judgment. Specifically, the detection unit SU has a function of taking a still picture and detecting a given action, in regard to one of the to-be-observed persons Ob to which the detection unit SU is assigned, wherein, it is operable, when it detects a given action (i.e., when it judges that a given action has been performed), to transmit the monitoring information including the still image taken when the judgment of the given action was made, and the judgment result.

The one of the to-be-observed persons Ob to which the detection unit SU is assigned means the to-be-observed person Ob-1 for the detection unit SU-1, the to-be-observed person Ob-2 for the detection unit SU-2, the to-be-observed person Ob-3 for the detection unit SU-3, and the to-be-observed person Ob-4 for the detection unit SU-4.

The detection unit SU is capable of discriminatingly judging two or more types of actions, as the given action. For example, it is capable of discriminatingly judging the actions described based on FIG. 4, i.e., four types of actions: "sitting-up"; "out-of-bed"; "fall-over/fall-off'; and "micro body motion abnormality". A way to judge these actions has already been described briefly.

A destination of the monitoring information is the management server unit SV. The management server unit SV is operable to receive and store the monitoring information, and concurrently transmit the received monitoring information to the stationary terminal unit SP and all of the mobile terminal units TA.

First of all, the feature 1 of the to-be-observed person monitoring system MS according to this embodiment will be described. Referring to FIG. 2, when the TA communication IF section 43 (communication section) receives new monitoring information via the network NW in a state in which the display processing part 4121 causes the TA display section 46 to display a monitoring information screen, the display processing part 4121 is operable to cause the TA display section 46 to display a monitoring information screen generated based on the new monitoring information received by the TA communication IF section 43, in place of the monitoring information screen currently displayed on the TA display section 46.

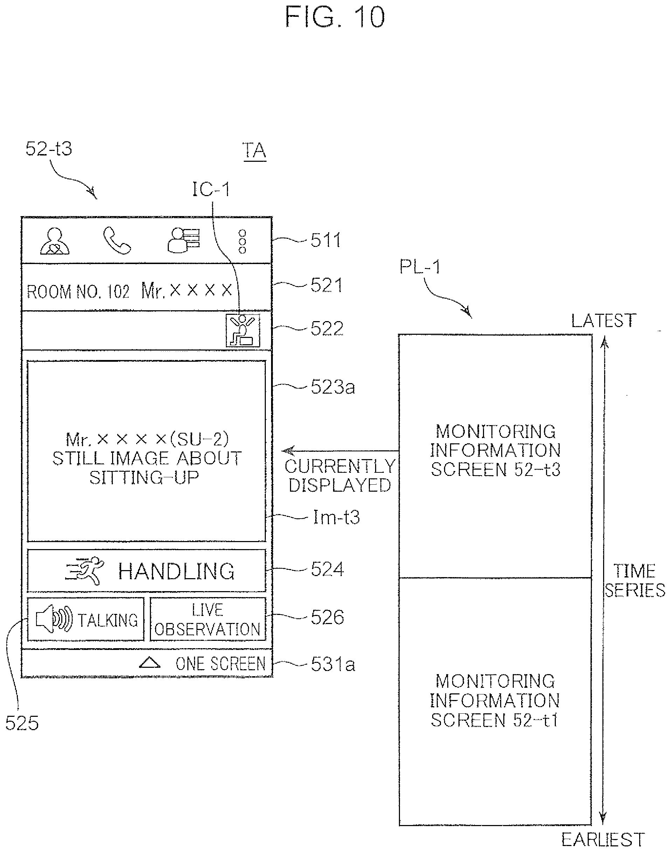

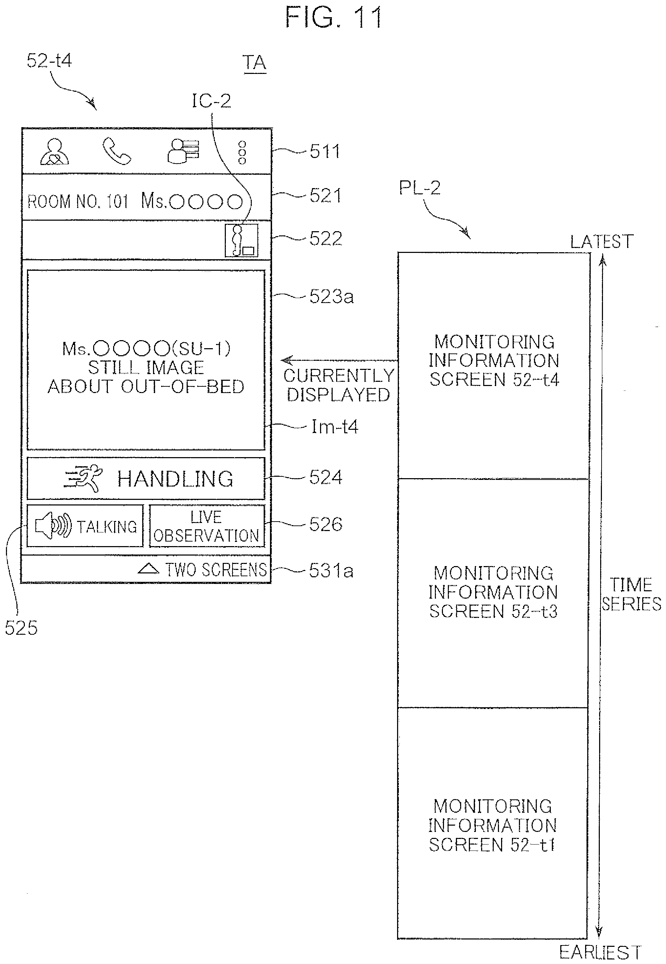

The feature 1 will be described in detail. The display processing part 4121 is operable to generate a monitoring information screen including the judgment result, based on the monitoring information and to cause the TA display section 46 to display the monitoring information screen. The judgment results are denoted, respectively, by the icons IC described based on FIG. 4. FIG. 10 is a diagram depicting one example of a monitoring information screen 52-t3 currently displayed on the TA display section 46. FIG. 11 is a diagram depicting one example of a monitoring information screen 52-t4 displayed on the TA display section 46, in place of the monitoring information screen 52-t3 depicted in FIG. 10.

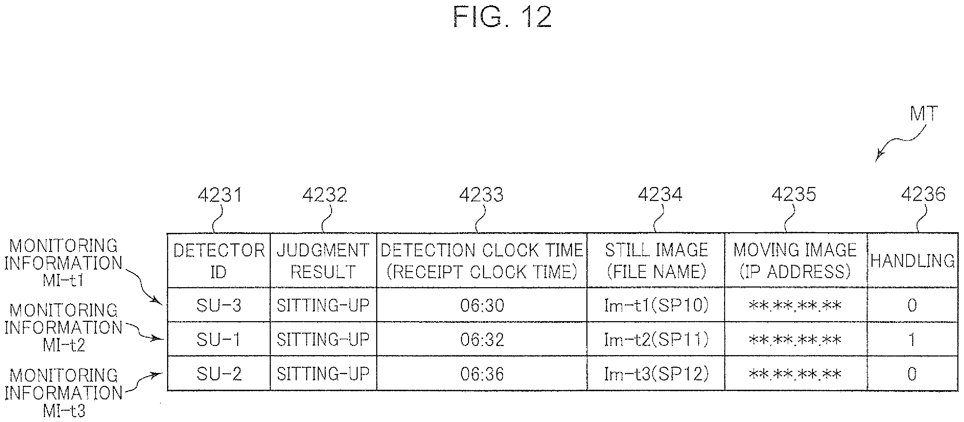

Assume that, at present, information presented in a monitoring information table MT depicted in FIG. 12 is stored in the monitoring information storage section 422 of each of the mobile terminal units TA, and a monitoring information screen 52-t3 depicted in FIG. 10 is displayed on the TA display section 46 of each of the mobile terminal units TA. Two monitoring information screens 52-t3, 52-t1 are time-serially coupled together to form a plane PL-1. The plane PL-1 is stored in the display screen storage section 421 (FIG. 2). The monitoring information table MT has already been described based on FIG. 3A. The monitoring information screen 52-t3 is a screen generated using the monitoring information screen MI-t3. Monitoring information MI-t3 is the latest monitoring information at this time. The term "monitoring information" means the record described based on FIG. 3A.

Referring to FIGS. 10 and 12, a to-be-observed person name specified by the detector ID included in the monitoring information MI-t3 is displayed in the to-be-observed person name area 521. The sitting-up icon IC-1 denoting the judgment result included the monitoring information MI-t3 is displayed in the icon area 522. A still image Im-t3 included in the monitoring information MI-t3 is displayed in the image area 523a.

An additional monitoring information reception window 531a is formed, for example, as a bar-shaped window, and indicates that there is a monitoring information screen whose "handling" button has not yet been touched, among monitoring information screens older than a monitoring information screen currently displayed on the TA display section 46. More specifically, as mentioned above, the monitoring information screen 52-t3 generated based on the monitoring information MI-t3 is displayed on the TA display section 46 of each of the mobile terminal units TA. The TA communication IF section 43 of each of the mobile terminal units TA had received the monitoring information MI-t1 and monitoring information MI-t2 before when it received the monitoring information MI-t3. Thus, the monitoring information screen 52-t1 generated based on the monitoring information MI-t1 and a monitoring information screen (not depicted) generated based on the monitoring information MI-t2 are older than the monitoring information screen 52-t3.

As to the monitoring information MI-t1, the handling field 4236 is "0". This means that, in regard to the monitoring information screen 52-t1 generated based on the monitoring information MI-t1, the "handling" button 524 has not yet been touched in any of the mobile terminal units TA. On the other hand, as to the monitoring information MI-t2, the handling field 4236 is "1". This means that, in regard to the monitoring information screen (not depicted) generated based on the monitoring information MI-t2, the "handling" button 524 has been touched in any one of the mobile terminal units TA.

The additional monitoring information reception window 531a indicates that there is a monitoring information screen generated based on monitoring information whose handling field is "0", among monitoring information screens older than a monitoring information screen currently displayed on the TA display section 46, and the number of such monitoring information screens. In FIG. 10, the additional monitoring information reception window 531a indicates that there is one monitoring information screen (monitoring information screen 52-t1). The monitoring information screen 52-t1 and the monitoring information screen 52-t3 are time-serially coupled together to form a plane. In this state, the monitoring information screen 52-t1 and the monitoring information screen 52-t3 are stored in the display screen storage section 421 (FIG. 2).

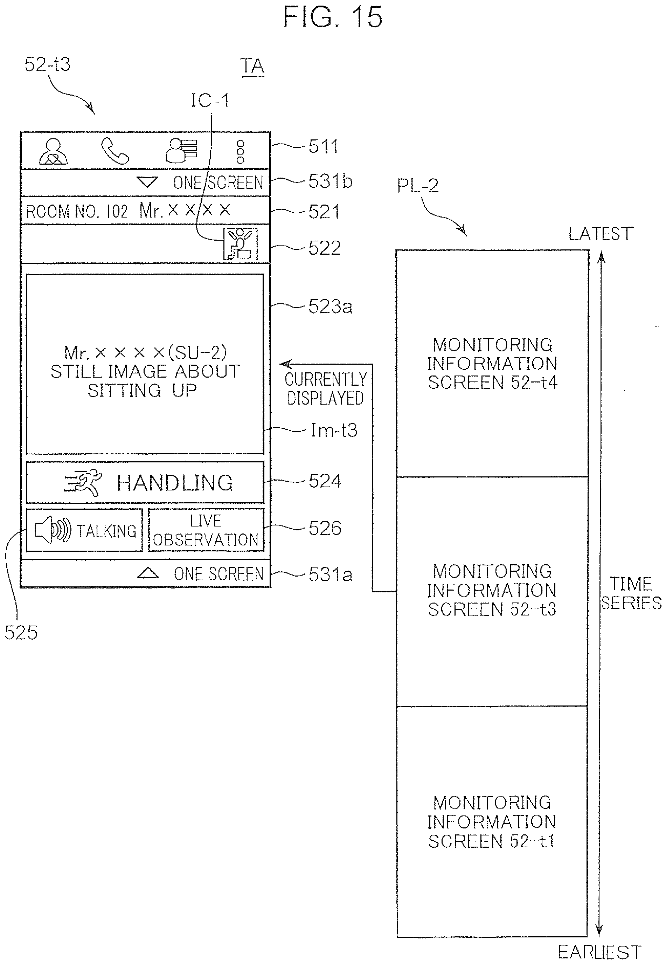

When there is no monitoring information screen whose "handling" button 524 has not yet been touched, among monitoring information screens older than a monitoring information screen currently displayed on the TA display section 46, the additional monitoring information reception window 531a is not included in the monitoring information screen currently displayed on the TA display section 46. Further, as described in detail later, when there is a monitoring information screen whose "handling" button 524 has not yet been touched, among monitoring information screens newer than a monitoring information screen currently displayed on the TA display section 46, an additional monitoring information reception window 531b is included in the monitoring information screen currently displayed on the TA display section 46 (FIG. 15).

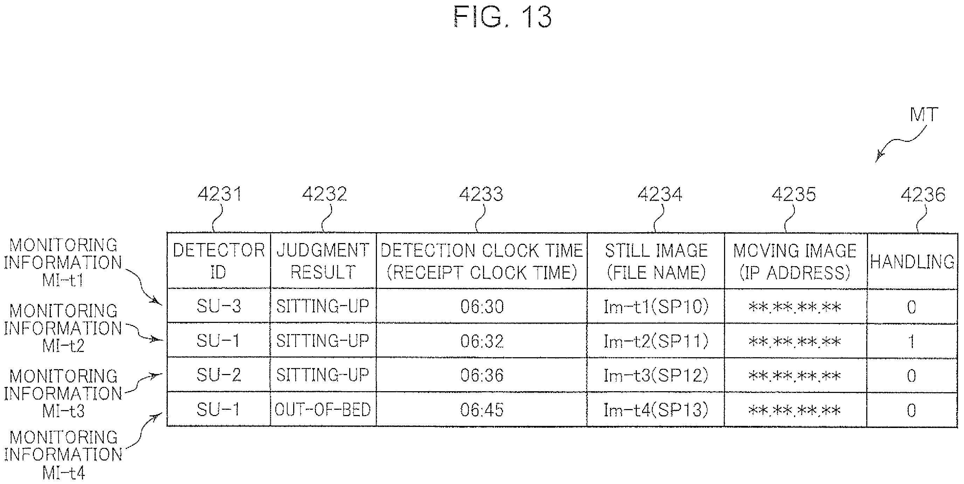

Referring to FIGS. 1, 2 and 10, assume that, for example, the detection unit SU-1 transmits monitoring information MI-t4 to the management server unit SV in a state in which the monitoring information screen 52-t3 is displayed on the TA display section 46 of each of the mobile terminal units TA. In this case, the management server unit SV simultaneously transmits (broadcasts) the received monitoring information MI-t4 to all of the mobile terminal units TA. The TA communication IF section 43 of each of the mobile terminal units TA receives the broadcasted monitoring information MI-t4, and the TA monitoring processing section 412 writes the monitoring information MI-t4 in the monitoring information table MT as depicted in FIG. 13 (In FIG. 5, the processing S2: YES, the processing S3: YES, the processing S4).

The display processing part 4121 of each of the mobile terminal units TA executes the processing S5 in FIG. 5. In this example, as depicted in FIG. 13, the detector ID included in the monitoring information MI-t4 is different from the detector ID includes in the monitoring information (monitoring information MI-t1, MI-t3) whose handling field 4236 is "0". Thus, the determination result in the processing S5 is YES.

The display processing part 4121 of each of the mobile terminal units TA executes the processing S6 in FIG. 5. In this example, as depicted in FIG. 13, in the monitoring information MI-t2 including the detector ID identical to the detector ID includes in the monitoring information MI-t4, the handling field 4236 is "1". Thus, the determination result in the processing S6 is NO, and the display processing part 4121 executes the processing S7. Since the determination result in the processing S5 is YES, the display processing part 4121 time-serially couples the monitoring information screen 52-t4 formed using the monitoring information MI-t4 and the already-existing monitoring information screens 52-t3, 52-t1 together to form a plane PL-2, as depicted in FIG. 11. The display processing part 4121 in each of the mobile terminal units TA switches a monitoring information screen to be displayed on the TA display section 46 from the monitoring information screen 52-t3 (currently-displayed monitoring information screen) depicted in FIG. 10 to the monitoring information screen 52-t4 (latest monitoring information screen) depicted in FIG. 11.

As depicted in FIG. 11, the time-serially coupled monitoring information screens 52-t1, 52-t3, 52-t4 are stored in the display screen storage section 421. The out-of-bed icon IC-2 denoting "out-of-bed" is displayed in the icon area 522. The additional monitoring information reception window 531a indicates that there are two monitoring information screens (monitoring information screens 52-t1, 52-t3).

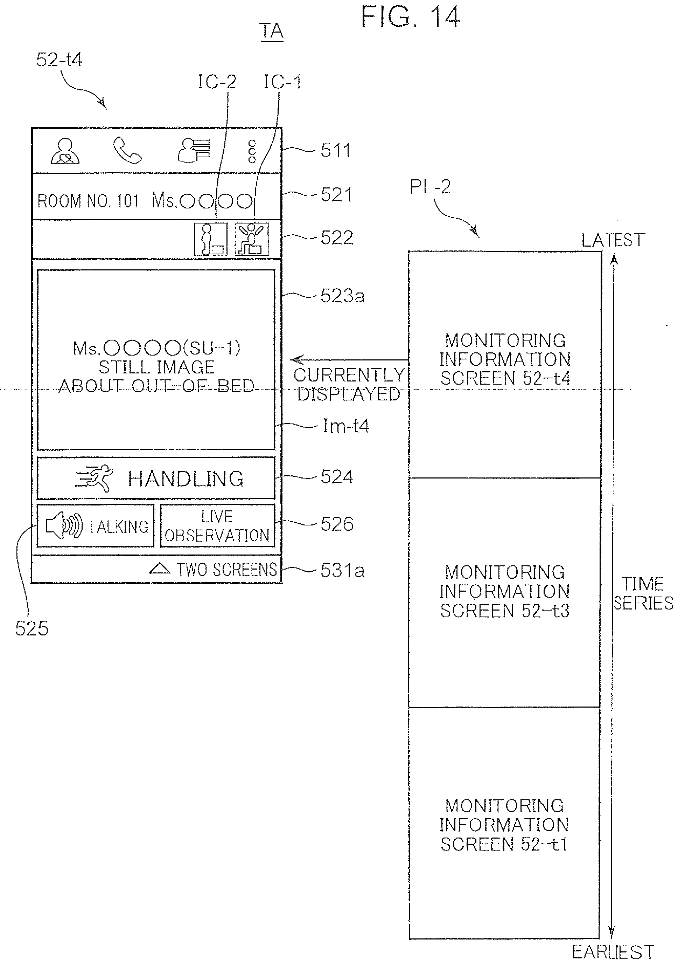

Supposing that the handling filed 4236 of the monitoring information MI-t2 depicted in FIG. 13 is "0", the "handling button" 524 in the monitoring information screen generated based on the monitoring information MI-t2 has not yet been touched in any of the mobile terminal units TA. In this case, the determination result in the processing S6 is YES, and and the display processing part 4121 executes the processing S8. Thus, the display processing part 4121 causes the TA display section 46 to display the monitoring information screen 52-t4 depicted in FIG. 14. A difference between the monitoring information screen 52-t4 depicted in FIG. 14 and the monitoring information screen 52-t4 depicted in FIG. 11 is that the sitting-up icon IC-1 is included in the monitoring information screen 52-t4 depicted in FIG. 14. The sitting-up icon IC-1 is the judgment result indicative of "sitting-up".

When the monitoring information MI-t4 is registered on the monitoring information table MT depicted in FIG. 12 in a state in which no monitoring information is registered thereon, the determination result in the processing S5 is NO, and the determination result in the processing S6 is NO. When the monitoring information MI-t4 is registered on the monitoring information table MT depicted in FIG. 12 in a state in which only the monitoring information MI-t2 is registered thereon (supposing that the handling filed 4236 is "0"), the determination result in the processing S5 is NO, and the determination result in the processing S6 is YES.

As described above, the feature 1 focuses on a point that, when the mobile terminal units TA receives new monitoring information in a state in which the mobile terminal units TA displays thereon a monitoring information screen, the received monitoring information is the latest monitoring information and thus it is desirable to display the content thereof as soon as possible. Particularly, in the case where the judgment result included in the new monitoring information is critical (e.g., fall-over or fall-off of the to-be-observed person Ob), the necessity becomes higher.

In the feature 1, the display processing part 4121 is operable, when the TA communication IF section 43 receives new monitoring information in a state in which the display processing part 4121 causes the TA display section 46 to display the monitoring information screen 52-t3 (FIG. 10) thereon, to cause the TA display section 46 to display thereon the monitoring information screen 52-t4 (FIG. 11) generated based on the new monitoring information, in place of the monitoring information screen 52-t3 currently displayed on the TA display section 46. Thus, in the feature 1, when new monitoring information arrives in a state in which a monitoring information screen is displayed, it is possible to prevent a user of the mobile terminal unit TA (i.e., monitorer) from overlooking the judgment result included in the new monitoring information.

Referring to FIG. 11, in the feature 1, the still image Im-t4 taken when the detection unit SU judged a given action of the to-be-observed person Ob is included in the monitoring information screen 52-t4. This allows the user of of the mobile terminal unit TA (monitorer) to check the state of the to-be-observed person Ob by the image. Further, in the feature 1, the judgment result is indicated by the icon IC, so that it is possible to reduce a size (surface area) of a display area (icon area 522) for the judgment result. This makes it possible to increase a size (surface area) of a display area (display area 523a) for a still image.

Next, the feature 2 of the to-be-observed person monitoring system MS according to this embodiment will be described. The display processing part 4121 is operable to switchably display the monitoring information screens 52-t1, 52-t3, 52-t4 time-serially coupled together as depicted in FIG. 11, on the TA display section 46. Referring to FIG. 2, the TA input section 45 is one example of a manual input section. The manual input section is configured to allow input based on a first manipulation and input based on a second manipulation different from the first manipulation to be performed thereon. The first manipulation is a manipulation of flicking a monitoring information screen displayed on the TA display section 46 downwardly. The second manipulation is a manipulation of flicking a monitoring information screen displayed on the TA display section 46 upwardly.

The display screen storage section 421 is configured to store therein a plurality of monitoring information screens (monitoring information screens 52-t1, 52-t3, 52-t4) each generated based on a respective one of a plurality of pieces of monitoring information (monitoring information MI-t1, MI-t3, MI-t4) including new monitoring information (monitoring information MI-t4) received by the TA communication IF section 43, in a state in which the plurality of monitoring information screens are time-serially arranged in order from latest to earliest in terms of timing when each of the plurality of pieces of the monitoring information is received by the TA communication IF section 43, in such a manner that the monitoring information screen (monitoring information screen 52-t4) generated based on the new monitoring information (monitoring information MI-t4) is positioned at a head of the plurality of monitoring information screens.

When a user of the mobile terminal unit TA flicks the monitoring information screen 52-t4 displayed on the TA display section 46 upwardly (second manipulation), the TA input section 45 accepts the flick. In response to this, the display processing part 4121 switches the monitoring information screen 52-t4 being displayed on the TA display 46 to the monitoring information screen 52-t3 next to and on the earliest (oldest) side with respect to the monitoring information screen 52-t4. This state is depicted in FIG. 15. FIG. 15 is a diagram depicting the monitoring information screen 52-t3 switched by flicking the monitoring information screen 52-t4 depicted in FIG. 11 upwardly.

In the state depicting in FIG. 15, when the user of the mobile terminal unit TA flicks the monitoring information screen 52-t3 displayed on the TA display section 46 upwardly (second manipulation), the TA input section 45 accepts the flick. In response to this, the display processing part 4121 switches the monitoring information screen 52-t3 being displayed on the TA display 46 to the monitoring information screen 52-t1 next to and on the earliest (oldest) side with respect to the monitoring information screen 52-t3 (this operation is not depicted). On the other hand, in the state depicting in FIG. 15, when the user flicks the monitoring information screen 52-t3 displayed on the TA display section 46 downwardly (first manipulation), the TA input section 45 accepts the flick. In response to this, the display processing part 4121 switches the monitoring information screen 52-t3 being displayed on the TA display 46 to the monitoring information screen 52-t4 next to and on the latest (newest) side with respect to the monitoring information screen 52-t3 (FIG. 11).

In this way, the display processing part 4121 is operable, in response to input to the TA input section 45 (manual input section), to switchably display a plurality of monitoring information screens (monitoring information screens 52-t1, 52-t3, 52-t4) stored in the display screen storage section 421. The display processing part 4121 is operable, when input based on downward flick (first manipulation) is performed on the TA input section 45, to read, from the display screen storage section 421, the monitoring information screen next to and on the latest side with respect to the monitoring information screen currently displayed on the TA display section 46, and cause the TA display section 46 to display the read monitoring information screen thereon. In other words, the display processing part 4121 is operable to cause the TA display section 46 to display the monitoring information screen next to and on the time-serially latest side with respect to the monitoring information screen being displayed on the TA display section 46. In one specific example, the display processing part 4121 is operable to switch from the monitoring information screen 52-t3 depicted in FIG. 15 to the monitoring information screen 52-t4 depicted in FIG. 11.

On the other hand, when input based on upward flick (second manipulation) is performed on the TA input section 45, the display processing part 4121 is operable to read, from the display screen storage section 421, the monitoring information screen next to and on the earliest side with respect to the monitoring information screen currently displayed on the TA display section 421, and cause the TA display section 46 to display the read monitoring information screen thereon. In other words, the display processing part 4121 is operable to cause the TA display section 46 to display the monitoring information screen next to and on the time-serially earliest side with respect to the monitoring information screen being displayed on the TA display section 46. In one specific example, the display processing part 4121 is operable to switch from the monitoring information screen 52-t4 depicted in FIG. 11 to the monitoring information screen 52-t3 depicted in FIG. 15.

As above, in the feature 2, the plurality of monitoring information screens 52-t1, 52-t3, 52-t4 are stored in the display screen storage section 421 in a state in which they are time-serially arranged in order of timing when each of a plurality of pieces of monitoring information as a basis of the monitoring information screens is received by the TA communication IF section 43, and the display processing part 4121 is capable of causing the TA display section 46 to switchably display the plurality of monitoring information screens sequentially along the time-series arrangement thereof.

In this embodiment, a direction of flick on the monitoring information screen is set in the upward-downward direction. Alternatively, it may be set to a direction slightly oblique to a rightward-leftward direction. Further, the first and second manipulations are not limited to flick, but may be any other suitable manipulation such as scroll or drag.

The feature 3 of the to-be-observed person monitoring system MS according to this embodiment will be described. The display processing part 4121 is operable, when input based on a third manipulation different from the downward flick (first manipulation) and the upward flick (second manipulation) is performed on the TA input section 45, to perform the following operations (a) and (b).

In the operation (a), the display processing part 4121 deletes, from the TA display section 46, a monitoring information screen currently displayed on the TA display section 46.

In the operation (b), the display processing part 4121 erases the monitoring information screen deleted from the TA display section 46, among the plurality of monitoring information screens stored in the display screen storage section 42.

Specifically, referring to FIG. 15, a user of the mobile terminal unit TA touches the "handling" button 524 included in the monitoring information screen 52-t3 (third manipulation), the display processing part 4121 is operable to delete the monitoring information screen 52-t3 from the TA display section 46, and erase the monitoring information screen 52-t3 stored in the display screen storage section 421. The display processing part 4121 is operable to time-serially couple the monitoring information screens (the monitoring information screens 52-t1, 52-t4) remaining in the display screen storage section 421 to form a new plane. Thus, target monitoring information screens to be switched by flick are the monitoring information screens 52-t1, 52-t4.

As described in connection with the feature 2, the display processing part 4121 is capable of causing the TA display section 46 to switchably display the plurality of monitoring information screens stored in the display screen storage section 421, sequentially along the time-series arrangement thereof. However, in the case where there are a large number of monitoring information screens between the monitoring information screen currently displayed on the TA display section 46 and a monitoring information screen to be displayed on the TA display section 46 next, it needs to take a lot of time for switching to the monitoring information screen to be displayed on the TA display section 46 next. Therefore, in the feature 3, when the "handling" button 524 is touched (third operation) in a state in which a certain monitoring information screen is displayed on the TA display section 46, the display processing part 4121 is operable, on an assumption that the certain monitoring information screen is deemed as an unnecessary monitoring information screen, to delete the certain monitoring information screen from the TA display section 46, and erase the certain monitoring information screen from the display screen storage section 421.

The feature 4 of the to-be-observed person monitoring system MS according to this embodiment will be described. When the feature 3 is implemented in any one of the plurality of mobile terminal units TA depicted in FIG. 1, the display processing part 4121 in each of the remaining mobile terminal units TA is operable to perform the above operations (a) and (b).

Specifically, the TA communication IF section 43 in each of the plurality of mobile terminal units TA receives monitoring information simultaneously notified from the management server unit SV to the plurality of mobile terminal units TA. Then, the display processing part 4121 in each of the plurality of mobile terminal units TA causes the TA display section 46 to display a monitoring information screen (e.g., the monitoring information screen 52-t4 depicted in FIG. 11) generated based on new monitoring information (e.g., the monitoring information IM-t4 depicted in FIG. 13) received by the TA communication IF section 43, in place of a monitoring information screen (e.g., the monitoring information screen 52-t3 depicted in FIG. 10) currently displayed on the TA display section 46. In this state, when, in any one of the plurality of mobile terminal units TA, the "handling" button 524 included in the monitoring information screen 52-t4 is touched (third operation), the display processing part 4121 in each of the remaining mobile terminal units TA performs the operations (a) and (b). That is, the display processing part 4121 deletes the monitoring information screen 52-t4 from the TA display section 46, and erases the monitoring information screen 52-t4 stored in the display screen storage section 421.

As described in connection with the feature 3, an unnecessary monitoring information screen stored in the display screen storage section 421 is likely to give rise to a need to take a lot of time for switching to a monitoring information screen to be displayed on the TA display section 46 next. Therefore, in response to touching the "handling" button 524 (third operation), a monitoring information screen which has become unnecessary is deleted from the TA display section 46, and further erased from the display screen storage section 421. In the feature 4 of the to-be-observed person monitoring system MS according to this embodiment, on an assumption that the unnecessary monitoring information screen is also unnecessary in the remaining mobile terminal units TA, the display processing part 4121 in each of the remaining mobile terminal units TA is operable to delete the unnecessary monitoring information screen from the TA display section 46, and erase the unnecessary monitoring information screen from the display screen storage section 421. This makes it possible to share information about the unnecessary monitoring information screen among the plurality of mobile terminal units TA.

The feature 5 of the to-be-observed person monitoring system MS according to this embodiment will be described. The display processing part 4121 is operable, when it causes the TA display section 46 to display thereon a monitoring information screen including a critical judgment result, to prevent the monitoring information screen from being switched to a monitoring information screen including a non-critical judgment result. Specifically, for example, assume that a predetermined action is defined as "fall-over/fall-off' of the to-be-observed person, and an action other than the predetermined action is defined as "sitting-up" of the to-be-observed person. Further, assume that a judgment result of "fall-over/fall-off' is defined as the critical judgment result, and a judgment result of "sitting-up" is defined as the non-critical judgment result.

As a way to judge "fall-over/fall-off' and "sitting-up", there are various techniques. One example thereof will be briefly described here. For example, the detection unit SU depicted in FIG. 1 is operable to extract a head image and a bed image from a taken still image. The detection unit SU preliminarily stores therein first data indicative of an area of a given region including an area of a head image of the to-be-observed person Ob when he/she sits up on a bed, and second data indicative of an area of a given region including an area of a head image of the to-be-observed person Ob when he/she lies on a floor.

When the extracted head image overlaps the extracted bed image, and an area of the extracted head image falls within the range of the area indicated by the first data, the detection unit SU is operable to judge that this state is "sitting-up". On the other hand, when an area of the extracted head image falls within the range of the area indicated by the second data, the detection unit SU is operable to judge that this state is "fall-over/fall-off'.

Assume a state in which the display processing part 4121 causes the TA display section 46 to display thereon a monitoring information screen generated based on monitoring information including a judgment result indicating that a first to-be-observed person (e.g., Ms.oooo) among the plurality of to-be-observed persons Ob falls over or falls off from a bed. In this state, when the TA communication IF section 43 receives new monitoring information including a judgment result indicating that a second to-be-observed person (e.g., Mr. × × × ×) different from the first to-be-observed person sits up on a bed, the display processing part 4121 is operable to cause the TA display section 46 to continue to display thereon the monitoring information screen currently displayed thereon (monitoring information screen including the judgment result indicative of "fall-over/fall-off' of Ms.○○○○) without switching.

As above, in the feature 5, in a situation where the display processing part 4121 cause the TA display section 46 to display thereon the monitoring information screen generated based on the monitoring information including as a judgment result, "fall-over/fall-off' of the first to-be-observed person, the case where the TA communication IF section 43 receives monitoring information including, as a judgment result, "sitting-up" of the second to-be-observed person different from the first to-be-observed person is determined as an exception. In this case, the display processing part 4121 is operable to cause the TA display section 46 to continue to display thereon the currently-displayed monitoring information screen (in which the judgment result is "fall-over/fall-off' of the first to-be-observed person) without switching. Therefore, in the feature 5, it is possible to, when a monitoring information screen including a critical judgment result (fall-over/fall-off) is displayed on the TA display section 46, prevent the monitoring information screen from being switched to a monitoring information screen including a non-critical judgment result (sitting-up").

The feature 6 of the to-be-observed person monitoring system MS according to this embodiment will be described. The display processing part 4121 is operable, when the TA communication IF section 43 receives new monitoring information including a critical judgment result in a state in which the display processing part 4121 causes the TA display section 46 to display thereon a monitoring information screen including a critical judgment result, to switch the monitoring information screen to a monitoring information screen based on the new monitoring information. Specifically, assume that the display processing part 4121 causes the TA display section 46 to display thereon a monitoring information screen generated based on monitoring information including a judgment result indicative of "fall-over/fall-off' of the first to-be-observed person. In this state, when the TA communication IF section 43 receives new monitoring information including a judgment result indicative of "fall-over/fall-off' of the second to-be-observed person, the display processing part 4121 is operable to cause the TA display section 46 to display thereon a monitoring information screen generated based on the new monitoring information, in place of the monitoring information screen displayed on the TA display section 46.

A user of the mobile terminal unit TA can view only a monitoring information screen currently displayed on the TA display section 46. Thus, as long as a critical judgment result is included in the currently-displayed monitoring information screen, the user can recognize the critical judgment result. However, even if the TA communication IF section 43 simply receives new monitoring information including a critical judgment result, the user cannot recognize the critical judgment result included in the new monitoring information. Therefore, in the feature 6, when the TA communication IF section 43 receives new monitoring information including a critical judgment result, the display processing part 4121 is operable to cause the TA display section 46 to display thereon a monitoring information screen generated based on the new monitoring information, even when a monitoring information screen currently displayed on the TA display section 46 includes a critical judgment result.

Next, first to third modifications of the above embodiment will be described. As described in connection with FIG. 5, in the operation of displaying a monitoring information screen on the mobile terminal unit TA, the display processing part 4121 is operable to assign one monitoring information screen to each of the to-be-observed persons Ob. Specifically, referring to FIGS. 11 and 13, in the case where, in regard to one to-be-observed person Ob (in this example, Ms.oooo), the plurality of pieces of monitoring information MI-t2, MI-t4 are stored in the monitoring information storage section 422, the display processing part 4121 is operable to generate the monitoring information screen 52-t4 based on the latest monitoring information MI-t4 of the to-be-observed person Ob.

The monitoring information screen 52-t4 has the judgment result (in this example, the out-of-bed icon IC-2 denoting "out-of-bed") and the still image Im-t4 each included in the latest monitoring information MI-t4 of the to-be-observed person Ob. In one MI-t2 of the remaining pieces of monitoring information of the to-be-observed person Ob, the handling field is "1". Supposing that it is "0", the display processing part 4121 is operable to cause the TA display section 46 to display thereon the monitoring information screen 52-t4 depicted in FOG. 14. The monitoring information screen 52-t4 depicted in FOG. 14 has the sitting-up icon IC-1 denoting the judgment result included in the monitoring information MI-t2, in addition to the out-of-bed icon IC-2.

In the first to third modifications, the display processing part 4121 is operable, when there are a plurality of pieces of monitoring information per to-be-observed person Ob, to assign a plurality of monitoring information screens to the to-be-observed person Ob.

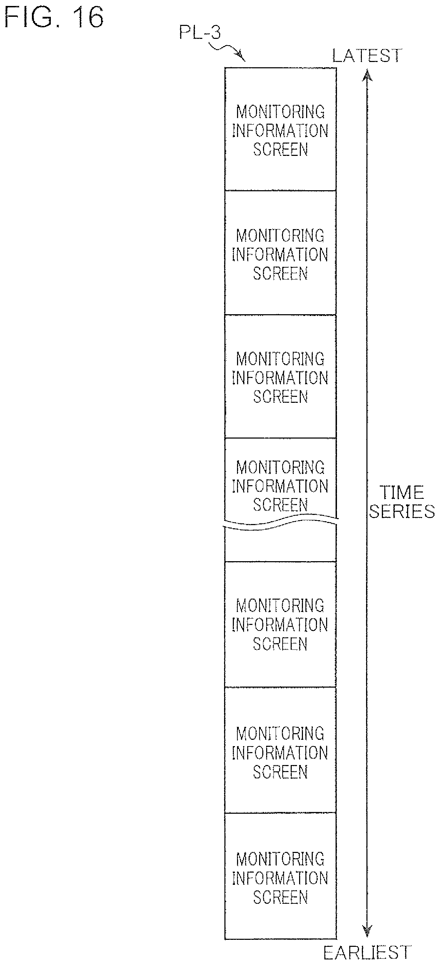

First of all, the first modification will be described. FIG. 16 is a diagram depicting one example of a plane PL-3 created in the first modification. The display processing part 4121 is operable to generate a plurality of monitoring information screens for all of a plurality of pieces of monitoring information included in the monitoring information table MT (FIG. 3), and time-serially couple the monitoring information screens together to form a plane PL-3. The operation of the mobile terminal unit TA when a user flicks the monitoring information screen displayed on the TA display section 46 is the same as that in the above embodiment. That is, when the user flicks the monitoring information screen displayed on the TA display section 46 downwardly, the display processing part 4121 is operable to cause the TA display section 46 to display the monitoring information screen next to and on the time-serially latest side with respect to the monitoring information screen. On the other hand, when the user flicks the monitoring information screen displayed on the TA display section 46 upwardly, the display processing part 4121 is operable to cause the TA display section 46 to display the monitoring information screen next to and on the time-serially earliest side with respect to the monitoring information screen.

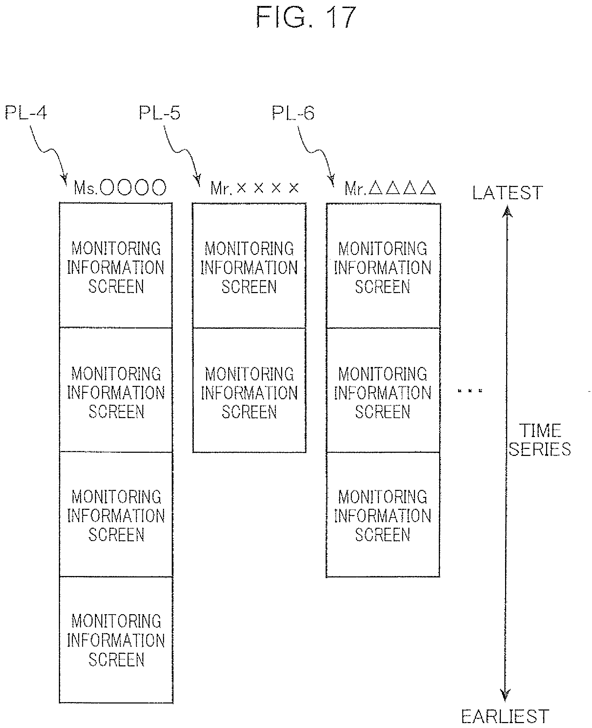

In the second modification, a plane PL is formed with respect to each of the plurality of to-be-observed persons. FIG. 17 is a diagram depicting one example of planes PL created in the second modification. The display processing part 4121 is operable to generate a plurality of monitoring information screens for all of a plurality of pieces of monitoring information of Ms.oooo included in the monitoring information table MT (FIG. 3), and time-serially couple these monitoring information screens together to form a plane PL-4. In regard to the remaining to-be-observed persons (Mr. ××××, Mr. Δ Δ Δ Δ, and others), the display processing part 4121 is operable to form, respectively, a plane PL-5, a plane PL-6 and others, in the same manner.

When a user of the mobile terminal unit TA depicted in FIG. 2 manipulates the TA input section 45 to perform an input for displaying a monitoring information screen corresponding to a desired one of the to-be-observed persons, the display processing part 4121 is operable to cause the TA display section 46 to display the monitoring information screen corresponding to the desired to-be-observed person. For example, when input for displaying a monitoring information screen corresponding to Ms.oooo is performed, the display processing part 4121 is operable to cause the TA display section 46 to display the monitoring information screen corresponding to Ms.oooo. The operation of the mobile terminal unit TA when a user flicks the monitoring information screen displayed on the TA display section 46 is the same as that in the above embodiment. For example, in a state in which the display processing part 4121 causes the TA display section 46 to display a monitoring information screen corresponding to Ms.○○○○, when the user flicks the monitoring information screen, the monitoring information screen is switched along the plane PL-4.

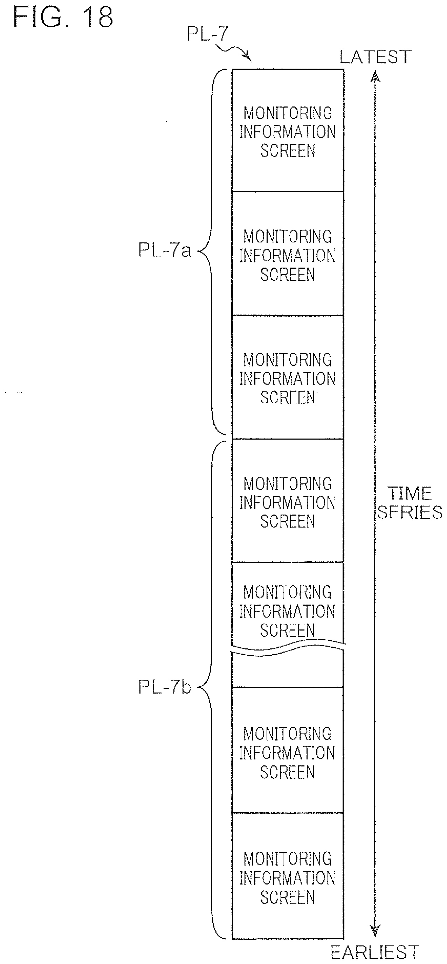

FIG. 18 is a diagram depicting one example of a plane PL-7 created in the third modification. The display processing part 4121 is operable to generate a plurality of monitoring information screens for all of a plurality of pieces of monitoring information included in the monitoring information table MT (FIG. 3), and divide these monitoring information screens into a first group of monitoring information screens each including a determination result indicating that the to-be-observed person Ob perform the predetermined action (e.g., "fall-over/fall-off'), and a second group of the remaining monitoring information screens. Then, the display processing part 4121 is operable to time-serially couple the first group of monitoring information screens together to form a plane PL-7a. Then, the display processing part 4121 is operable to time-serially couple the second group of monitoring information screens together to form a plane PL-7b. Further, the display processing part 4121 is operable to couple the plane PL-7a and the plane PL-7b, such that the plane PL-7a is disposed on the latest side with respect to the plane PL-7b, to thereby form a plane P-7.

In the first to third modifications, the display processing part 4121 may be configured to avoid generating any monitoring information screen using monitoring information whose handling filed 4236 is "1".

Although the features 1 to 6 of the to-be-observed person monitoring system MS according to the above embodiment and the first to third modifications have been described based on an example where the mobile terminal unit TA is employed as a monitoring information screen display device, the management server unit SV or the stationary terminal unit SP depicted in FIG. 1 may be configured as the monitoring information screen display device.

### (Outline of Embodiment)

According to a first aspect of the above embodiment, there is provided a monitoring information screen display device for use in a to-be-observed person monitoring system equipped with a sensor device which transmits monitoring information including a result of judgment when it judges that a to-be-observed person has performed a given action. The monitoring information screen display device comprises: a communication section which receives the monitoring information via a network; a display section; and a display processing section which generates a monitoring information screen including the judgment result, based on the monitoring information received by the communication section, and causes the display section to display the monitoring information screen thereon, wherein the display processing section is operable, when the communication section receives new monitoring information in a state in which the display processing section causes the display section to display the monitoring information screen thereon, to cause the display section to display thereon another monitoring information screen generated based on the new monitoring information received by the communication section, in place of the monitoring information screen currently displayed on the display section.

When the communication section receives new monitoring information in a state in which the display processing section causes the display section to display a monitoring information screen, the display processing section is operable to cause the display section to display a monitoring information screen generated based on the new monitoring information, in place of the monitoring information screen currently displayed on the TA display section 46. Thus, in the monitoring information screen display device according to the first aspect of the above embodiment, when new monitoring information arrives in a state in which a monitoring information screen is displayed, it is possible to prevent a monitorer from overlooking the judgment result included in the new monitoring information.

Preferably, the above monitoring information screen display device further comprises: a manual input section which allows input based on a first manipulation and input based on a second manipulation different from the first manipulation to be performed thereon; and a storage section which stores therein a plurality of the monitoring information screens each generated based on a respective one of a plurality of pieces of the monitoring information including the new monitoring information received by the communication section, in a state in which the plurality of monitoring information screens are time-serially arranged in order from latest to earliest in terms of timing when each of the plurality of pieces of the monitoring information is received by the communication section, in such a manner that the monitoring information screen generated based on the new monitoring information is positioned at a head of the plurality of monitoring information screens, wherein the display processing section is operable, when the input based on the first manipulation is performed on the manual input section, to read, from the storage section, the monitoring information screen next to and on a latest side with respect to the monitoring information screen currently displayed on the display section, and cause the display section to display the read monitoring information screen thereon, and, when the input based on the second manipulation is performed on the manual input section, to read, from the storage section, the monitoring information screen next to and on an earliest side with respect to the monitoring information screen currently displayed on the display section, and cause the display section to display the read monitoring information screen thereon.

According to this feature, the plurality of monitoring information screens are stored in the storage section in a state in which they are time-serially arranged in order of timing when each of a plurality of pieces of monitoring information as a basis of the monitoring information screens is received by the communication section, and the display processing section is capable of causing the display section to switchably display the plurality of monitoring information screens sequentially along the time-series arrangement thereof.

Preferably, in the above monitoring information screen display device, the display processing section is operable, when input based on a third manipulation different from the first manipulation and the second manipulation is performed on the manual input section, to (a) delete, from the display section, the monitoring information screen currently displayed on the display section, and (b) erase the monitoring information screen deleted from the display section, among the plurality of monitoring information screens stored in the storage section.

The display processing section is capable of causing the display section to switchably display the plurality of monitoring information screens sequentially along the time-series arrangement thereof. However, in the case where there are a large number of monitoring information screens between the monitoring information screen currently displayed on the display section and a monitoring information screen to be displayed on the display section next, it needs to take a lot of time for switching to the monitoring information screen to be displayed next. Therefore, the display processing section is operable, in response to the third manipulation, to delete an unnecessary monitoring information screen from the display section, and erase the unnecessary monitoring information screen from the storage section.

Preferably, in the above monitoring information screen display device, the to-be-observed person monitoring system includes a plurality of the sensor devices assigned, respectively, to a plurality of the to-be-observed persons, wherein each of the plurality of sensor devices is capable of discriminatingly judging two or more types of actions as the given action, and wherein, in a state in which the display processing section causes the display section to display thereon the monitoring information screen generated based on the monitoring information including the judgment result indicating that a first to-be-observed person among the plurality of to-be-observed persons has performed a predetermined action among the two or more types of actions, the display processing section is operable, when the communication section receives new monitoring information including the judgment result indicating that a second to-be-observed person different from the first to-be-observed person has performed an action other than the predetermined action, to cause the display section to continue to display thereon the monitoring information screen currently displayed thereon without switching.

For example, assume that the predetermined action is defined as "fall-over/fall-off" of the to-be-observed person, and an action other than the predetermined action is defined as "sitting-up" of the to-be-observed person. In a situation where the display processing section cause the display section to display thereon the monitoring information screen generated based on the monitoring information including as a judgment result, "fall-over/fall-off' of the first to-be-observed person, the case where the communication section receives monitoring information including, as a judgment result, "sitting-up" of the second to-be-observed person different from the first to-be-observed person is determined as an exception. In this case, the display processing section is operable to cause the display section to continue to display thereon the currently-displayed monitoring information screen (in which the judgment result is "fall-over/fall-off' of the first to-be-observed person) without switching. Therefore, according to the above feature, it is possible to, when a monitoring information screen including a critical judgment result (fall-over/fall-off) is displayed on the display section, prevent the monitoring information screen from being switched to a monitoring information screen including a non-critical judgment result (sitting-up").

Preferably, in the above monitoring information screen display device, in a state in which the display processing section causes the display section to display thereon the monitoring information screen generated based on the monitoring information including the judgment result indicating that the first to-be-observed person has performed the predetermined action, the display processing section is operable, when the communication section receives new monitoring information including the judgment result indicating that the second to-be-observed person has performed the predetermined action, to cause the display section to display thereon the monitoring information screen generated based on the new monitoring information, in place of the monitoring information screen displayed on the display section.

A user of the monitoring information screen display device can view only a monitoring information screen currently displayed on the display section. Thus, as long as a critical judgment result is included in the currently-displayed monitoring information screen, the user can recognize the critical judgment result. However, even if the communication section simply receives new monitoring information including a critical judgment result, the user cannot recognize the critical judgment result included in the new monitoring information. Therefore, according to the above feature, when the communication section receives new monitoring information including a critical judgment result, the display processing section is operable to cause the display section to display thereon a monitoring information screen generated based on the new monitoring information, even when a monitoring information screen currently displayed on the display section includes a critical judgment result.

Preferably, in the above monitoring information screen display device, the sensor device has a function of taking a still image about the to-be-observed person, wherein the sensor device is operable, when it judges that the to-be-observed person has performed the given action, to transmit the monitoring information including the still image taken at a time when the judgment about the given action was made, and a result of the judgment, and wherein the display processing section is operable to generate the monitoring information screen including the still picture and an icon representing the judgment result, and cause the display section to display the monitoring information screen thereon.

According to this feature, the still image taken when the sensor device judged the given action of the to-be-observed person is included in the monitoring information screen, so that a user of the monitoring information screen display device (monitorer) can check the state of the to-be-observed person by the image. Further, according to this feature, the judgment result is indicated by the icon IC, so that it is possible to reduce a size (surface area) of a display area for the judgment result.

According to a second aspect of the above embodiment, there is provided a to-be-observed person monitoring system which comprises: a sensor device configured to, when it judges that a to-be-observed person has performed a given action, transmit monitoring information including a result of the judgment; and the above monitoring information screen display device.

The to-be-observed person monitoring system according to the second aspect of the above embodiment has the same functions/advantageous effects as those of the monitoring information screen display device according to the first aspect of the above embodiment.

Preferably, the above to-be-observed person monitoring system comprises a plurality of the monitoring information screen display devices, wherein, in a state in which, in each of the plurality of monitoring information screen display devices, the communication section receives the monitoring information notified concurrently to the plurality of monitoring information screen display devices, and the display processing section causes the display section to display the monitoring information screen generated based on the new monitoring information received by the communication section, in place of the monitoring information screen currently displayed on the display section, when the input based on the third manipulation is performed in one of the plurality of monitoring information screen display devices, the display processing section in each of the remaining monitoring information screen display devices is operable to perform the operation (a) and the operation (b).

As mentioned above, an unnecessary monitoring information screen (e.g., a monitoring information screen whose "handling" button has already been touched) stored in the storage section is likely to give rise to a need to take a lot of time for switching to a monitoring information screen to be displayed on the display section next. Therefore, in response to the third operation, a monitoring information screen which has become unnecessary is deleted from the display section, and further erased from the storage section. According to this feature, on an assumption that the unnecessary monitoring information screen is also unnecessary in the monitoring information screen display devices, the display processing section in each of the remaining monitoring information screen display devices is operable to delete the unnecessary monitoring information screen from the display section, and erase the unnecessary monitoring information screen from the storage section. This makes it possible to share information about the unnecessary monitoring information screen among the plurality of monitoring information screen display devices.

According to a third aspect of the above embodiment, there is provided a monitoring information screen display method for use in a to-be-observed person monitoring system equipped with a sensor device configured to, when it judges that a to-be-observed person has performed a given action, transmit monitoring information including a result of the judgment. The monitoring information screen display method comprises: a first step of receiving the monitoring information via a network; a second step of generating a monitoring information screen including the judgment result, based on the monitoring information received in the first step, and causing a display section to display the monitoring information screen thereon; a third step of determining whether or not new monitoring information has been received, in a state in which the monitoring information screen generated in the second step is displayed on the display section; and a fourth step of, when it is determined, in the third step, that new monitoring information has been received, causing the display section to display another monitoring information screen generated based on the new monitoring information received in the third step, in place of the monitoring information screen currently displayed on the display section.

The monitoring information screen display method according to the third aspect of the above embodiment has the same functions/advantageous effects as those of the monitoring information screen display device according to the first aspect of the above embodiment.

This application is based on Japanese Patent Application Serial No. 2015-158175 filed in Japan Patent Office on August 10, 2015, the contents of which are hereby incorporated by reference.

While the present invention has been described appropriately and fully by way of the embodiment as above with reference to the drawings in order to express the present invention, it should be appreciated that anyone skilled in the art can readily change and/or modify the embodiment described above. It is therefore understood that a changed embodiment or a modified embodiment implemented by anyone skilled in the art is enclosed within the scope of the appended claims unless the changed embodiment or the modified embodiment is of a level that deviates from the scope of the appended claims.

### INDUSTRIAL APPLICABILITY

The present invention can provide a to-be-observed person monitoring system, a monitoring information screen display device and a monitoring information screen display method.

## Claims

1. A monitoring information screen display device for use in a to-be-observed person monitoring system equipped with a sensor device which transmits monitoring information including a result of judgment when it judges that a to-be-observed person has performed a given action, the monitoring information screen display device comprising:
a communication section which receives the monitoring information via a network;
a display section; and
a display processing section which generates a monitoring information screen including the judgment result, based on the monitoring information received by the communication section, and causes the display section to display the monitoring information screen thereon,
wherein the display processing section is operable, when the communication section receives new monitoring information in a state in which the display processing section causes the display section to display the monitoring information screen thereon, to cause the display section to display thereon another monitoring information screen generated based on the new monitoring information received by the communication section, in place of the monitoring information screen currently displayed on the display section.

2. The monitoring information screen display device as recited in claim 1, which further comprises:
a manual input section which allows input based on a first manipulation and input based on a second manipulation different from the first manipulation to be performed thereon; and
a storage section which stores therein a plurality of the monitoring information screens each generated based on a respective one of a plurality of pieces of the monitoring information including the new monitoring information received by the communication section, in a state in which the plurality of monitoring information screens are time-serially arranged in order from latest to earliest in terms of timing when each of the plurality of pieces of the monitoring information is received by the communication section, in such a manner that the monitoring information screen generated based on the new monitoring information is positioned at a head of the plurality of monitoring information screens,
wherein the display processing section is operable, when the input based on the first manipulation is performed on the manual input section, to read, from the storage section, the monitoring information screen next to and on a latest side with respect to the monitoring information screen currently displayed on the display section, and cause the display section to display the read monitoring information screen thereon, and, when the input based on the second manipulation is performed on the manual input section, to read, from the storage section, the monitoring information screen next to and on an earliest side with respect to the monitoring information screen currently displayed on the display section, and cause the display section to display the read monitoring information screen thereon.

3. The monitoring information screen display device as recited in claim 2, wherein the display processing section is operable, when input based on a third manipulation different from the first manipulation and the second manipulation is performed on the manual input section, to (a) delete, from the display section, the monitoring information screen currently displayed on the display section, and (b) erase the monitoring information screen deleted from the display section, among the plurality of monitoring information screens stored in the storage section.

4. The monitoring information screen display device as recited in any one of claims 1 to 3, wherein the to-be-observed person monitoring system includes a plurality of the sensor devices assigned, respectively, to a plurality of the to-be-observed persons,
wherein each of the plurality of sensor devices is capable of discriminatingly judging two or more types of actions as the given action,
and wherein, in a state in which the display processing section causes the display section to display thereon the monitoring information screen generated based on the monitoring information including the judgment result indicating that a first to-be-observed person among the plurality of to-be-observed persons has performed a predetermined action among the two or more types of actions, the display processing section is operable, when the communication section receives new monitoring information including the judgment result indicating that a second to-be-observed person different from the first to-be-observed person has performed an action other than the predetermined action, to cause the display section to continue to display thereon the monitoring information screen currently displayed thereon without switching.

5. The monitoring information screen display device as recited in claim 4, wherein in a state in which the display processing section causes the display section to display thereon the monitoring information screen generated based on the monitoring information including the judgment result indicating that the first to-be-observed person has performed the predetermined action, the display processing section is operable, when the communication section receives new monitoring information including the judgment result indicating that the second to-be-observed person has performed the predetermined action, to cause the display section to display thereon the monitoring information screen generated based on the new monitoring information, in place of the monitoring information screen displayed on the display section.

6. The monitoring information screen display device as recited in claim 4 or 5, wherein each of the plurality of sensor device is operable to judge, as the predetermined action, fall-over or fall-off of a respective one of the to-be-observed persons.

7. The monitoring information screen display device as recited in any one of claims 1 to 6, wherein the sensor device has a function of taking a still image about the to-be-observed person, wherein the sensor device is operable, when it judges that the to-be-observed person has performed the given action, to transmit the monitoring information including the still image taken at a time when the judgment about the given action was made, and a result of the judgment, and wherein the display processing section is operable to generate the monitoring information screen including the still picture and an icon representing the judgment result, and cause the display section to display the monitoring information screen thereon.

8. A to-be-observed person monitoring system comprising:
a sensor device which transmits monitoring information including a result of judgment when it judges that a to-be-observed person has performed a given action; and
the monitoring information screen display device as recited in any one of claims 1 to 7.

9. The to-be-observed person monitoring system as recited in claim 8, which comprises a plurality of the monitoring information screen display devices as recited in claim 3, wherein, in a state in which, in each of the plurality of monitoring information screen display devices, the communication section receives the monitoring information notified concurrently to the plurality of monitoring information screen display devices, and the display processing section causes the display section to display the monitoring information screen generated based on the new monitoring information received by the communication section, in place of the monitoring information screen currently displayed on the display section, when the input based on the third manipulation is performed in one of the plurality of monitoring information screen display devices, the display processing section in each of the remaining monitoring information screen display devices is operable to perform the operation (a) and the operation (b).

10. A monitoring information screen display method for use in a to-be-observed person monitoring system equipped with a sensor device which transmits monitoring information including a result of judgment when it judges that a to-be-observed person has performed a given action, the monitoring information screen display method comprising:
a first step of receiving the monitoring information via a network;
a second step of generating a monitoring information screen including the judgment result, based on the monitoring information received in the first step, and causing a display section to display the monitoring information screen thereon;
a third step of determining whether or not new monitoring information has been received, in a state in which the monitoring information screen generated in the second step is displayed on the display section; and
a fourth step of, when it is determined, in the third step, that new monitoring information has been received, causing the display section to display another monitoring information screen generated based on the new monitoring information received in the third step, in place of the monitoring information screen currently displayed on the display section.
